(19) Europäisches Patentamt / European Patent Office / Office européen des brevets

(11) **EP 3 304 183 B1**

(12) **EUROPÄISCHE PATENTSCHRIFT**

(45) Veröffentlichungstag und Bekanntmachung des Hinweises auf die Patenterteilung:
**12.12.2018 Patentblatt 2018/50**

(51) Int Cl.:
*G02C 13/00* (2006.01)   *A61B 3/08* (2006.01)
*A61B 3/11* (2006.01)

(21) Anmeldenummer: **17740331.8**

(22) Anmeldetag: **05.07.2017**

(86) Internationale Anmeldenummer:
**PCT/EP2017/066763**

(87) Internationale Veröffentlichungsnummer:
**WO 2018/015154 (25.01.2018 Gazette 2018/04)**

(54) **FERN-DURCHBLICKPUNKT-BESTIMMUNG FÜR EIN BRILLENGLAS**

DETERMINATION OF FAR-VISION POINT ON SPECTACLES LENS

DÉTERMINATION DU POINT DE VISION DE LOIN SUR DES LENTILLES DE LUNETTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.07.2016 DE 102016113374**

(43) Veröffentlichungstag der Anmeldung:
**11.04.2018 Patentblatt 2018/15**

(73) Patentinhaber: **Carl Zeiss Vision International GmbH**
**73430 Aalen (DE)**

(72) Erfinder:
• **CABEZA-GUILLÈN, Jesús-Miguel**
**73434 Aalen (DE)**

• **MANI, Subhashini**
**74357 Bönnigheim (DE)**
• **GAMPERLING, Michael**
**89340 Leipheim (DE)**

(74) Vertreter: **Gauss, Nikolai et al**
**Pfiz/Gauss Patentanwälte PartmbB**
**Tübingerstraße 26**
**70178 Stuttgart (DE)**

(56) Entgegenhaltungen:
**DE-A1-102014 200 637   US-A1- 2010 128 220**
**US-A1- 2010 195 045   US-A1- 2015 198 822**

**Beschreibung**

Die Erfindung ist in den Ansprüche definiert. Sie betrifft ein Verfahren zum Bestimmen eines Fern-Durchblickpunkts auf einem in einer Brillenfassung aufnehmbaren Brillenglas, die eine Fassungsebene hat, bei dem ein in einer Bildebene liegendes Bild wenigstens eines Abschnitts einer einem Proband aufgesetzten Brillenfassung mit einer eine optische Achse aufweisenden Kamera erfasst wird, während der Proband mit einer die Fassungsebene durchsetzenden Blickrichtung wenigstens eines Auges in die Kamera blickt, bei dem ein auf die Lage der Bildebene bezogener Vorneigungswinkel $\alpha'$ der Brillenfassung ermittelt wird, der entsprechend dem erfassten Neigungswinkel $\gamma$ der Bildebene gegenüber der vertikalen Richtung zu einem auf die vertikale Richtung bezogenen Vorneigungswinkel $\alpha$ korrigiert wird, bei dem ein von der optischen Achse der Kamera mit einer zu der Abstandslinie der Pupillen der Augen des Probanden senkrechten Ebene gebildeter Kopfdrehwinkel $\beta$ des Kopfs des Probanden ermittelt wird, bei dem der Kopfdrehwinkel $\beta$ entsprechend dem erfassten Neigungswinkel $\gamma$ der Bildebene gegenüber der vertikalen Richtung zu einem einer horizontalen Ausrichtung der optischen Achse der Kamera entsprechenden korrigierten Kopfdrehwinkel $\beta'$ korrigiert wird, bei dem der Fern-Durchblickpunkt durch Analysieren des in der Bildebene liegenden Bilds unter Berücksichtigung des einer horizontalen Ausrichtung der optischen Achse der Kamera entsprechenden korrigierten Kopfdrehwinkels $\beta'$ bestimmt wird und bei dem der Schnittpunkt der Blickrichtung mit der Fassungsebene mittels Bildauswertung bestimmt wird. Darüber hinaus betrifft die Erfindung auch ein System für das Bestimmen eines solchen Fern-Durchblickpunkts sowie ein Computerprogramm.

Aus der WO 2015/107063 A1 sind ein derartiges Verfahren und System sowie ein derartiges Computerprogramm bekannt.

Um die Brillengläser korrekt in eine Brillenfassung einzupassen, ist es erforderlich, sogenannte Anpassparameter zu bestimmen, damit die optischen Zentren der Gläser mit den visuellen Achsen der entsprechenden Augen in Deckung gebracht werden können, um so z. B. die Information über den Pupillenabstand und die Information über die Höhe der Pupillen in Bezug auf die Brillenfassung zu kennen. Darüber hinaus ist es wichtig, die Höhe der optischen Zentren der Brillengläser in Bezug auf den unteren oder oberen Rand der Brillenfassung zu bestimmen, in das die Brillengläser eingesetzt werden.

Anpassparameter können z. B. bestimmt werden, indem sich ein Optiker und ein Proband gegenüberstehen oder gegenübersitzen, wobei der Proband die Fassung seiner Wahl mit einer darin eingefassten Stützscheibe aufsetzt. Der Proband wird gebeten, in die Ferne zu blicken, und der Optiker zeichnet dann nach Augenschein auf der Scheibe oder auf einer Kontaktstrichfolie mit einem Kreuz den Durchblick an, wie er ihn beim gegenseitigen Anblick erkennt. Dieses Kreuz (Zentrierkreuz) bestimmt dann die Lage des optischen Mittelpunktes des in die Fassung einzusetzenden Brillenglases. Dieses Verfahren wird für jedes der Augen eines Probanden einzeln durchgeführt. Der Abstand der auf diese Weise ermittelten Zentrierkreuze ist der Pupillenabstand PD.

In der WO 01/84222 A1 ist ein System für das Bestimmen von Anpassparametern für Brillengläser beschrieben, das eine an einer Säule höhenverstellbar aufgenommene digitale Kamera enthält, deren Objektiv zusammen mit einem Spiegel und einer Lichtquelle im Bereich der Frontfläche des Gehäuses angeordnet ist. Dieses System umfasst einen mit der digitalen Videokamera verbundenen Computer, der aus dem Bild eines Probanden mit einer Brillenfassung und mit einem an der Brillenfassung festgelegten Messbügel Anpassparameter für die Brillenfassung mittels Bildauswertung bestimmt. Zum Bestimmen der Anpassparameter wird hier der Proband gebeten, direkt in die digitale Kamera zu blicken, wobei die optische Achse der Kamera zu der Blickrichtung des Probanden parallel sein muss. Alternativ hierzu kann auch ein Verlagern der Kamera vorgesehen sein, um sicherzustellen, dass die Bildebene der Kamera zu der Ebene des Messbügels parallel ist und das Bild der Augen des Probanden dann in der Bildebene der Kamera zu deren optischer Achse zentriert ist.

In der US 2010/0195045 A1 ist ein Verfahren zum Bestimmen von Brillenglas-Anpassparametern beschrieben, bei dem aus dem mit einer Kamera erfassten Bild eines Probanden, der eine Brille mit einem daran festgelegten Messbügel trägt, der gesuchte Anpassparameter mittels Bildanalyse bestimmt wird. Hier wird der Proband gebeten, auf eine LED zu blicken, die an der Kamera angebracht ist. Die Kamera enthält einen Neigungssensor, um damit den Neigungswinkel der optischen Achse der Kamera zur Horizontalen zu bestimmen. Wenn der mittels des Neigungssensors erfasste Neigungswinkel einen Schwellwert übersteigt, gibt die Kamera ein Warnsignal ab. Auf dieses Weise wird sichergestellt, dass die optische Achse der Kamera bei der Bildaufnahme die Fassungsebene der Brillenfassung immer in etwa senkrecht schneidet. Damit wird erreicht, dass ein durch den Abstand der Augen des Probanden von der Fassungsebene hervorgerufener Parallaxefehler bei der Anpassparameterbestimmung vernachlässigbar ist. Für den Fall, dass die Fassungsebene zu der Bildebene der Kamera nicht parallel ist, wird in der US 2010/0195045 A1 eine rechnerische Bildkorrektur vorgeschlagen, um eine durch diese Aufnahmesituation hervorgerufene Bildverzerrung damit auszugleichen.

Aufgabe der Erfindung ist es, bei einem Probanden das exakte Bestimmen des Fern-Durchblickpunkts auf Brillengläsern in einer Brillenfassung unter Berücksichtigung der habituellen Kopfhaltung bzw. Körperhaltung zu ermöglichen.

Zur Lösung dieser Aufgabe werden die in den Patentansprüchen 1 und 11 angegebenen Merkmalskombinationen vorgeschlagen. Vorteilhafte Ausgestaltungen und Weiterbildungen der Erfindung ergeben sich aus den abhängigen Ansprüchen.

Die Erfindung ermöglicht es insbesondere, die Koordinaten des Fern-Durchblickpunkts auf Brillengläsern in einem zu der Brillenfassung ortsfeste Koordinatensystem anzugeben, d. h. in einem Fassungskoordinatensystem. Der Erfindung liegt der Gedanke zugrunde, dass zum Ermitteln der Anpassparameter für Brillengläser in eine Brillenfassung mittels Analysieren von Bildern einer einem Proband aufgesetzten Brillenfassung nicht zwingend eine fest in dem Raum montierte Kamera eingesetzt werden muss, sondern dass sich diese Anpassparameter grundsätzlich auch ermitteln lassen, indem Bilder einer mit den Händen gehaltenen Kamera ausgewertet werden, die z. B. in einen Tabletcomputer integriert ist. Die Erfinder haben allerdings erkannt, dass die auf diese Weise ermittelten Anpassparameter oftmals von den Anpassparametern abweichen, die mittels Analysieren entsprechender Bilder bestimmt werden, die mit einer im Raum fest montierten Kamera erfasst werden, wie dies z. B. in der WO 01/84222 A1 beschrieben ist.

Verfahren zum Bestimmen Anpassungsparameter gibt es auch in US2010/128220, US2010/195045, US2015/198822 und DE102014200637.

In umfangreichen Versuchen haben die Erfinder herausgefunden, dass für den Fehler, der bei dem Bestimmen von Brillenglas-Anpassparametern für Brillengläser, die in eine Brillenfassung aufgenommen werden sollen, mittels Analysieren von Bildern der einem Proband aufgesetzten Brillenfassung auftreten kann, wenn die entsprechenden Bilder mit einer Kamera aufgenommen werden, die nicht fest montiert ist, sondern die mit den Händen gehalten wird, der Neigungswinkel der Bildebene der Kamera zur vertikalen Richtung beim Aufnehmen der Bilder maßgeblich ist.

Insbesondere haben die Erfinder herausgefunden, dass sich der Einfluss des Neigungswinkels der Bildebene der Kamera zur vertikalen Richtung anders als das Verkippen der Kamera um die optische Achse eines Kamera-Objektivsystems oder das Verschwenken der Kamera um eine in der vertikalen Richtung verlaufende Achse mittels Bildauswertung nicht ohne weiteres kompensieren lässt.

Eine Idee der Erfindung ist es deshalb insbesondere, mit einem Neigungssensor, wie er in Tabletcomputern mit einer Kamera oder in Smartphones mit einer Kamera wie z. B. dem IPAD® oder dem IPHONE® regelmäßig integriert ist, die Neigung der Kamera-Bildebene zu der vertikalen Richtung zu erfassen und dann bei einem Bestimmen des Fern-Durchblickpunkts und weiterer Anpassparameter für Brillengläser mittels Bildauswertung von Bildern einer mit einem Messbügel versehenen Brillenfassung, die einem Proband aufgesetzt ist, zusammen mit der Lage wenigstens eines auf dem Proband angeordneten Referenzpunkts relativ zu der Brillenfassung und/oder der Kamera-Bildebene zu berücksichtigen.

Wird nämlich z. B. ein Tabletcomputer bei der Aufnahme nicht exakt vertikal gehalten, so weicht der von der Kamera beobachtete Vorneigungswinkel $\alpha'$ für Brillengläser in der Brillenfassung von dem tatsächlichen Vorneigungswinkel $\alpha$ dieser Brillengläser ab, so dass dann z. B. beim Berechnen von Zentrierdaten mittels Bildauswertung aus solchen Aufnahmen sehr große Fehler auftreten können. Die berechneten Zentrierdaten, insbesondere die Durchblickhöhe des Probanden durch das Brillenglas, sind dann im Allgemeinen fehlerbehaftet und hängen davon ab, wie der Proband seinen Kopf gegenüber der Kamera orientiert, d. h. sowohl davon, wie weit er den Kopf gegenüber der Kamera anhebt oder absenkt, als auch davon, wie weit er den Kopf relativ zu der Kamera verdreht.

Die Erfindung schlägt deshalb zum Bestimmen eines Fern-Durchblickpunkts auf einem in einer Brillenfassung aufnehmbaren Brillenglas, die eine Fassungsebene hat, vor, dass ein in einer Bildebene liegendes Bild wenigstens eines Abschnitts einer einem Proband aufgesetzten Brillenfassung, das die Pupillen der Augen des Probanden enthält, mit einer eine optische Achse aufweisenden Kamera erfasst wird, während der Proband mit einer die Fassungsebene durchsetzenden Blickrichtung wenigstens eines Auges in die Kamera blickt. Bei dem erfindungsgemäßen Verfahren wird ein auf die Lage der Bildebene bezogener Vorneigungswinkel $\alpha'$ der Brillenfassung ermittelt, der entsprechend dem erfassten Neigungswinkel $\gamma$ der Bildebene gegenüber der vertikalen Richtung zu einem auf die vertikale Richtung bezogenen Vorneigungswinkel $\alpha$ korrigiert wird. Bei dem erfindungsgemäßen Verfahren wird auch ein von der optischen Achse der Kamera mit einer zu der Abstandslinie der Pupillen der Augen des Probanden senkrechten Ebene gebildeter Kopfdrehwinkel $\beta$ des Kopfs des Probanden ermittelt. Außerdem wird bei dem erfindungsgemäßen Verfahren der Kopfdrehwinkel $\beta$ entsprechend dem erfassten Neigungswinkel $\gamma$ der Bildebene gegenüber der vertikalen Richtung zu einem einer horizontalen Ausrichtung der optischen Achse der Kamera entsprechenden korrigierten Kopfdrehwinkel $\beta'$ korrigiert, und es wird der Fern-Durchblickpunkt durch Analysieren des in der Bildebene liegenden Bilds unter Berücksichtigung des einer horizontalen Ausrichtung der optischen Achse der Kamera entsprechenden korrigierten Kopfdrehwinkels $\beta'$ bestimmt. Der Schnittpunkt der Blickrichtung mit der Fassungsebene wird dann mittels Bildauswertung bestimmt.

Dabei wird der Fern-Durchblickpunkt als der Schnittpunkt einer in einer horizontalen Ebene liegenden virtuellen Blickrichtung mit der Fassungsebene bestimmt, wobei der Schnittpunkt der Blickrichtung mit der Fassungsebene mittels Bildauswertung bestimmt wird, und wobei die virtuelle Blickrichtung als die Richtung einer virtuellen Gerade bestimmt wird, die durch Drehen einer durch einen auf dem Auge angeordneten Referenzpunkt bekannter Lage in Bezug auf die Kamera oder der Brillenfassung und den Schnittpunkt der Blickrichtung festgelegten Geraden ermittelt wird. Hierfür wird diese Gerade zum einen in einem von dem Referenzunkt beabstandeten, innerhalb des Auges auf dieser Geraden liegenden virtuellen Drehpunkt um einen von dem korrigierten Kopfdrehwinkel ($\beta'$) abhängigen ersten Winkel ($\beta_{OD}''$,

$\beta_{OS}$") um eine zu der vertikalen Richtung parallele Achse gedreht. Zum anderen wird diese Gerade in dem virtuellen Drehpunkt um eine weitere Achse um einen von dem korrigierten Vorneigungswinkel ($\alpha$) abhängigen weiteren Winkel ($\alpha$") gedreht. Diese weitere Achse ist zu der Abstandslinie der Pupillen der Augen des Probanden parallel.

Für den von dem korrigierten Kopfdrehwinkel ($\beta'$) abhängigen ersten Winkel ($\beta_{OD}$") gilt dabei, wenn das Auge ein rechtes Auge des Probanden ist: $\tan \beta_{OD}$" = $(0,5 \times P_D \times \cos \beta' + YZ) / (D + HSA)$, und wenn das Auge 62 ein linkes Auge des Probanden 68 ist: $\tan \beta_{OS}$" = $(0,5 \times P_D \times \cos \beta' - YZ) / (D + HSA)$.

$P_D$ ist dabei die Pupillendistanz der Augen des Probanden, wobei $D + HSA$ der senkrechte Abstand der Kamera von einer vertikalen Ebene ist, in der sich die Abstandslinie der Pupillen der Augen befindet, und wobei YZ die Ablage des Schnittpunkts (Y) der Abstandslinie der Pupillen der Augen mit der senkrechten Projektion der optischen Achse der Kamera in eine horizontale Ebene ist, in der sich die Abstandlinie der Pupillen der Augen befindet, von dem Schnittpunk (Z) der Abstandslinie der Pupillen der Augen mit einer die Abstandslinie der Pupillen der Augen schneidenden vertikalen und zu der Fassungsebene senkrechten Ebene, in der die vertikale Symmetrieachse der Brillenfassung liegt. Unter der senkrechten Projektion einer Geraden bzw. einer Achse in eine Ebene wird dabei vorliegend eine Projektion der Achse bzw. Geraden in die Ebene in einer Richtung verstanden, die zu der Ebene senkrecht ist. Für den von dem korrigierten Vorneigungswinkel ($\alpha$) abhängigen weiteren Winkel ($\alpha$") gilt: $\alpha$" = $- (\gamma + \delta)$, wobei $\delta$ der Winkel ist, den die optische Achse der Kamera mit der senkrechten Projektion der Blickrichtung in eine vertikale Ebene bildet, in der die optische Achse der Kamera liegt.

Die Erfindung ermöglicht das Bestimmen wenigstens eines Anpassparameters, indem zunächst ein Fern-Durchblickpunkt bestimmt wird und dann aus dem Fern-Durchblickpunkt und aus dem erfassten Bild mittels Bildauswertung der wenigstens eine Anpassparameter.

Die Erfindung erreicht, dass bei der Anpassparameterbestimmung ein durch die Ablage der Augen des Probanden von der Brillenfassung bedingter Parallaxefehler und damit insbesondere die habituelle Kopfhaltung des Probanden berücksichtigt werden kann. Die mit dem erfindungsgemäßen Verfahren ermittelten Anpassparameter können dann insbesondere wiederspiegeln, dass das Führungsauge eines Probanden beim Betrachten eines Objekts dem Objekt zugewandt sein kann während das andere Auge des Probanden gegenüber dem Führungsauge relativ zu dem Objekt zurückgesetzt positioniert ist.

Zum Bestimmen des Fern-Durchblickpunkts und auch weiterer Anpassparameters mit dem erfindungsgemäßen Verfahren ist es nicht erforderlich, dass die Kamera in einer bestimmten Position oder Orientierung gehalten wird. Vielmehr genügt es, dass die Kamera das Gesicht des Probanden mit der von dem Proband getragenen Brillenfassung so erfasst, dass das Bild des Probanden die Pupillen beider Augen enthält, und außerdem solche Abschnitte der Brillenfassung, die es ermöglichen, bei einer bekannten Geometrie der Brillenfassung mittels Bildauswertung des mit der Kamera erfassten Bilds die Relativposition der Brillenfassung zu der Bildebene der Kamera zu ermitteln.

Der auf die Lage der Bildebene bezogene Vorneigungswinkel $\alpha'$ der Brillenfassung kann dann z. B. mittels Bildanalyse des erfassten Bilds des wenigstens einen Abschnitts der dem Proband aufgesetzten Brillenfassung ermittelt werden. Insbesondere ist es möglich, dass der von der optischen Achse der Kamera mit einer zu der Abstandslinie der Pupillen der Augen des Probanden senkrechten Ebene gebildete Kopfdrehwinkel $\beta$ des Kopfs des Probanden mittels Bildanalyse des erfassten Bilds des wenigstens einen Abschnitts der dem Proband aufgesetzten Brillenfassung ermittelt wird.

Eine besonders exakte Bestimmung des Fern-Durchblickpunkts und auch weiterer Anpassparameter ist allerdings möglich, indem das in der Bildebene liegende Bild zusammen mit zumindest drei zu der Brillenfassung ortsfesten Frontzielmarken erfasst wird, wobei zumindest eine der Frontzielmarken gegenüber den zumindest zwei anderen Frontzielmarken senkrecht zur Vorderseite räumlich versetzt angeordnet ist. Diese Frontzielmarken können z. B. an einem Messbügel ausgebildet sein, der an der Brillenfassung festgelegt ist. Die drei an der Vorderseite des Messbügels angeordneten Frontzielmarken definieren dann ein räumliches Koordinatensystem.

Bevorzugt gilt für den korrigierte Vorneigungswinkel $\alpha = \alpha' - \gamma$ und für den korrigierten Kopfdrehwinkel $\beta' = \beta / \cos (\alpha' - \gamma)$.

Vorteilhafter Weise wird das Bild der Brillenfassung in dem erfindungsgemäßen Verfahren mit einer digitalen Kamera erfasst, die einen die Neigung der Bildebene $\gamma$ um eine horizontale Achse erfassenden Neigungssensor enthält.

Dieser Neigungssensor kann z. B. ein Schwerkraftsensor sein, der die Richtung der Schwerkraft ermittelt. Alternativ hierzu kann der Neigungssensor auch als ein Sensor ausgebildet sein, der die Richtung des Erdmagnetfelds auswertet. Insbesondere kann der Neigungssensor auch ein sowohl die Richtung des Erdmagnetfelds als auch die Richtung der Schwerkraft auswertender Sensor sein, z. B. ein kombinierter Schwerkraft-/Magnetfeldsensor.

Das Bild des Abschnitts der dem Proband aufgesetzten Brillenfassung kann dabei z. B. erfasst werden, wenn an die Brillenfassung ein Messbügel angeschlossen ist, der eine Vorderseite mit zumindest drei Frontzielmarken für eine Messung des Vorneigungswinkels $\alpha$ der zu vermessenden Brillenfassung hat, wobei zumindest eine der Frontzielmarken gegenüber den zumindest zwei anderen Frontzielmarken senkrecht zur Vorderseite des Messbügels räumlich versetzt angeordnet ist.

Als Anpassparameter können so aus einem Bild wenigstens eines Abschnitts der dem Proband aufgesetzten Brillenfassung und dem Damit bestimmten Fern-Durchblickpunkts z. B. das Fassungsmaß (I, h, AzG), die Pupillendistanz ($P_D$, $z_R$, $z_L$), der Zentrierpunktabstand ($x_R$, $y_R$, $x_L$, $y_L$), die Fassungsvorneigung, die Fassungsscheibenwinkel und/oder der erforderliche Rohglasdurchmesser bestimmt werden.

Der Referenzpunkt ist bevorzugt der Hornhautscheitelpunkt des Auges des Probanden. Der Hornhautscheitelpunkt kann z. B. über eine reflexbasierte Zentrierung bestimmt werden. Es ist aber auch möglich, dass der Referenzpunkt eine Mitte der Pupille des Auges des Probanden ist. Diese Mitte der Pupille kann z. B. mittels Bildverarbeitung auf einem Computer ermittelt werden oder von einer Bedienperson, z. B. einem Augenoptiker, in einem Bild der Augen des Probanden bestimmt werden.

Die bekannte Lage des wenigstens einen auf den Proband angeordneten Referenzpunkts in dem durch die zumindest drei zu der Brillenfassung ortsfesten Frontzielmarken definierten Koordinatensystem kann z. B. durch Analysieren eines Bildes des Probanden von der Seite ermittelt werden, z. B. eines Bilds des Probanden, der einen Messbügel mit drei jeweils an den beiden Seiten des Messbügels angeordneten Zielmarken für das Bestimmen des Hornhautscheitelabstands und des Fassungsscheibenwinkels trägt, wie er in der DE 10 2004 063 981 B4 beschrieben ist. Insbesondere kann das analysierte Bild des Probanden ein mit der Kamera erfasstes Bild des Probanden von der Seite sein. Die an den beiden Seiten des Messbügels angeordneten Zielmarken sind dann in einem Winkel von im Wesentlichen 90° zu den Frontzielmarken angebracht. Sie ermöglichen das Kalibrieren eines Bildes des Probanden von der Seite und damit das Vermessen des Hornhautscheitelabstands auf der Grundlage eines solchen Bilds.

Zu bemerken ist allerdings, dass der Hornhautscheitelabstand (HSA) auch durch Vermessen des Probanden mittels eines sogenannten PD-Messstabes bestimmt werden kann, um ihn dann als eine bekannte Lage des wenigstens einen auf den Proband angeordneten Referenzpunkts in dem durch die zumindest drei zu der Brillenfassung definierten ortsfesten Frontzielmarken definierten Koordinatensystem in dem erfindungsgemäßen Verfahren zum Bestimmen wenigstens eines Anpassparameters für ein in einer Brillenfassung aufnehmbares Brillenglas zu berücksichtigen. Zu bemerken ist, dass in dem erfindungsgemäßen Verfahren der Hornhautscheitelabstand auch als ein Durchschnittswert für eine Vielzahl von Probanden aus einer Datenbank ausgelesen werden kann. Insbesondere ist zu bemerken, dass dieser Durchschnittswert z. B. ein Durchschnittswert für den Hornhautscheitelabstand einer bestimmten Population von Probanden oder bestimmter Subgruppen einer Population von Probanden sein kann.

Die Lage des virtuellen Drehpunkts auf der durch den Referenzpunkt bekannter Lage in Bezug auf die Kamera oder der Brillenfassung und den Schnittpunkt der Blickrichtung festgelegten Geraden kann z. B. durch Vorgeben eines festen Abstands (A) des virtuellen Drehpunkts von dem Referenzpunkt bestimmt werden. Die Pupillendistanz $P_D$ der Augen des Probanden kann z. B. mittels Bildanalyse eines mit der Kamera erfassten Bilds ermittelt werden, das die Pupillen der Augen des Probanden und wenigstens einen Abschnitt der dem Proband aufgesetzten Brillenfassung enthält. Der Winkel ($\delta$), den die optische Achse der Kamera mit der senkrechten Projektion der Blickrichtung in eine vertikale Ebene bildet, kann z. B. mittels Bildanalyse des erfassten Bilds des wenigstens einen Abschnitts der dem Proband aufgesetzten Brillenfassung ermittelt werden.

Die Ablage des Schnittpunkts (Y) der Abstandslinie der Pupillen der Augen mit der senkrechten Projektion der optischen Achse der Kamera in eine horizontale Ebene, in der sich die Abstandlinie der Pupillen der Augen befindet, von dem Schnittpunk (Z) der Abstandslinie der Pupillen der Augen mit einer die Abstandslinie der Pupillen der Augen schneidenden vertikalen und zu der Fassungsebene senkrechten Ebene, in der die vertikale Symmetrieachse der Brillenfassung liegt, kann ebenfalls mittels Bildanalyse eines mit der Kamera erfassten Bilds ermittelt werden, das die Pupillen der Augen des Probanden und wenigstens einen Abschnitt der dem Proband aufgesetzten Brillenfassung enthält.

Ein erfindungsgemäßes System zum Bestimmen eines Fern-Durchblickpunkts auf einem in einer Brillenfassung aufnehmbaren Brillenglas, die eine Fassungsebene hat, enthält eine Kamera, die eine Bildebene aufweist, mit einem die Neigung der Bildebene der Kamera um eine horizontale Achse erfassenden Neigungssensor. In dem System gibt es eine Rechnereinheit mit Mitteln für das Bestimmen eines auf die Lage der Bildebene bezogenen Vorneigungswinkels $\alpha$' der Brillenfassung, mit Mitteln für das Korrigieren des Vorneigungswinkels $\alpha$' der Brillenfassung entsprechend dem erfassten Neigungswinkel $\gamma$ der Bildebene gegenüber der vertikalen Richtung zu einem auf die vertikale Richtung bezogenen Vorneigungswinkel $\alpha$ mit Mitteln für das Bestimmen eines von der optischen Achse der Kamera mit einer zu der Abstandslinie der Pupillen der Augen des Probanden senkrechten Ebene gebildeten Kopfdrehwinkels $\beta$ des Kopfs des Probanden, und mit Mitteln für das Korrigieren des Kopfdrehwinkels $\beta$ entsprechend dem erfassten Neigungswinkel $\gamma$ der Bildebene gegenüber der vertikalen Richtung zu einem einer horizontalen Ausrichtung der optischen Achse der Kamera entsprechenden korrigierten Kopfdrehwinkel $\beta$'. Die Rechnereinheit enthält Mittel für das Bestimmen eines Schnittpunkts der Blickrichtung des Probanden mit der Fassungsebene. Die Rechnereinheit hat außerdem Mittel für das Bestimmen des Fern-Durchblickpunkts als der Schnittpunkt einer in einer horizontalen Ebene liegenden virtuellen Blickrichtung mit der Fassungsebene, durch welche die virtuelle Blickrichtung als die Richtung einer virtuellen Gerade bestimmt wird, die durch Drehen einer durch einen auf dem Auge angeordneten Referenzpunkt bekannter Lage in Bezug auf die Kamera oder der Brillenfassung und den Schnittpunkt der Blickrichtung festgelegten Geraden ermittelt wird, indem diese Gerade zum einen in einem von dem Referenzunkt beabstandeten, innerhalb des Auges auf dieser Geraden liegenden virtuellen Drehpunkt um einen von dem korrigierten Kopfdrehwinkel ($\beta$') abhängigen ersten Winkel ($\beta_{OD}$",

$\beta_{OS}$") um eine zu der vertikalen Richtung parallele Achse gedreht wird. Zum anderen wird diese Gerade in dem virtuellen Drehpunkt um eine weitere Achse um einen von dem korrigierten Vorneigungswinkel ($\alpha$ abhängigen weiteren Winkel ($\alpha$") gedreht. Diese weitere Achse ist zu der Abstandslinie der Pupillen der Augen des Probanden parallel. Für den von dem korrigierten Kopfdrehwinkel ($\beta$') abhängigen ersten Winkel ($\beta_{OD}$") gilt dabei, wenn das Auge ein rechtes Auge des Probanden ist: $\tan \beta_{OD}" = (0{,}5 \times P_D \times \cos \beta' + YZ) / (D + HSA)$, und wenn das Auge 62 ein linkes Auge des Probanden 68 ist: $\tan \beta_{OS}" = (0{,}5 \times P_D \times \cos \beta' - YZ) / (D + HSA)$. $P_D$ ist dabei die Pupillendistanz der Augen des Probanden. D + HSA ist der senkrechte Abstand der Kamera von einer vertikalen Ebene, in der sich die Abstandslinie der Pupillen der Augen befindet. YZ ist die Ablage des Schnittpunkts (Y) der Abstandslinie der Pupillen der Augen mit der senkrechten Projektion der optischen Achse der Kamera in eine horizontale Ebene, in der sich die Abstandlinie der Pupillen der Augen befindet, von dem Schnittpunk (Z) der Abstandslinie der Pupillen der Augen mit einer die Abstandslinie der Pupillen der Augen schneidenden vertikalen und zu der Fassungsebene senkrechten Ebene, in der die vertikale Symmetrieachse der Brillenfassung liegt. Für den von dem korrigierten Vorneigungswinkel ($\alpha$) abhängigen weiteren Winkel ($\alpha$") gilt: " = - ($\gamma$ + $\delta$), wobei $\delta$ der Winkel ist, den die optische Achse der Kamera mit der senkrechten Projektion der Blickrichtung in eine vertikale Ebene bildet, in der die optische Achse der Kamera liegt.

Die Mittel für das Bestimmen eines auf die Lage der Bildebene bezogenen Vorneigungswinkels $\alpha$' der Brillenfassung, die Mittel für das Korrigieren des Vorneigungswinkels $\alpha$' der Brillenfassung entsprechend dem erfassten Neigungswinkel $\gamma$ der Bildebene gegenüber der vertikalen Richtung zu einem auf die vertikale Richtung bezogenen Vorneigungswinkel $\alpha$ und die Mittel für das Bestimmen eines von der optischen Achse der Kamera mit einer zu der Abstandslinie der Pupillen der Augen des Probanden senkrechten Ebene gebildeten Kopfdrehwinkels $\beta$ des Kopfs des Probanden sowie die Mittel für das Korrigieren des Kopfdrehwinkels $\beta$ entsprechend dem erfassten Neigungswinkel $\gamma$ der Bildebene gegenüber der vertikalen Richtung zu einem einer horizontalen Ausrichtung der optischen Achse der Kamera entsprechenden korrigierten Kopfdrehwinkel $\beta$' und die Mittel für das Bestimmen des wenigstens einen Anpassparameters durch Analysieren des in der Bildebene liegenden Bilds unter Berücksichtigung des einer horizontalen Ausrichtung der optischen Achse der Kamera entsprechenden korrigierten Kopfdrehwinkels $\beta$' können als eine Rechnereinheit mit einem in die Rechnereinheit geladenen Computerprogramm ausgebildet sein. Entsprechendes gilt für die Mittel für das Bestimmen eines Schnittpunkts der Blickrichtung des Probanden mit der Fassungsebene und die Mittel für das Bestimmen des Fern-Durchblickpunkts.

Die Kamera kann in einen Tablet-Computer und/oder ein Mobiltelefon integriert sein, wobei die Rechnereinheit bevorzugt als ein mit dem Tablet-Computer und/oder dem Mobiltelefon verbundener Server ausgebildet ist. Auf diese Weise kann für das Auswerten der erfassten Bilder eine kurze Rechenzeit gewährleistet werden.

Indem der Tablet-Computer und/oder das Mobiltelefon mit dem Server drahtlos kommuniziert, können die Bilder eines Abschnitts einer einem Proband aufgesetzten Brillenfassung bei größtmöglicher Bewegungsfreiheit aufgenommen werden.

Das erfindungsgemäße Computerprogramm enthält insbesondere Programmcode für das Durchführen der nachfolgend angegebenen Verfahrensschritte in einem vorstehend beschriebenen Verfahren zum Bestimmen eines Fern-Durchblickpunkts und bevorzugt auch wenigstens eines Anpassparameters für ein in einer Brillenfassung aufnehmbares Brillenglas, wenn das Computerprogramm in der Rechnereinheit eines vorstehend beschriebenen Systems ausgeführt wird:

Erfassen eines in einer Bildebene liegenden Bilds wenigstens eines Abschnitts einer einem Proband aufgesetzten Brillenfassung, das die Pupillen der Augen des Probanden enthält, mit einer eine optische Achse aufweisenden Kamera, während der Proband mit einer die Fassungsebene durchsetzenden Blickrichtung wenigstens eines Auges in die Kamera blickt;

Ermitteln eines auf die Lage der Bildebene bezogener Vorneigungswinkels $\alpha$' der Brillenfassung;

Korrigieren dieses Vorneigungswinkels $\alpha$' entsprechend dem erfassten Neigungswinkel $\gamma$ der Bildebene gegenüber der vertikalen Richtung zu einem auf die vertikale Richtung bezogenen Vorneigungswinkel a;

Ermitteln eines von der optischen Achse der Kamera mit einer zu der Abstandslinie der Pupillen der Augen des Probanden senkrechten Ebene gebildeten Kopfdrehwinkels $\beta$ des Kopfs des Probanden;

Korrigieren des Kopfdrehwinkels $\beta$ entsprechend dem erfassten Neigungswinkel $\gamma$ der Bildebene gegenüber der vertikalen Richtung zu einem einer horizontalen Ausrichtung der optischen Achse der Kamera entsprechenden korrigierten Kopfdrehwinkel $\beta$';

Bestimmen des Fern-Durchblickpunkts durch Analysieren des in der Bildebene liegenden Bilds unter Berücksichtigung des einer horizontalen Ausrichtung der optischen Achse der Kamera entsprechenden korrigierten Kopfdrehwinkels $\beta$' wie folgt:

Bestimmen des Schnittpunkts der Blickrichtung mit der Fassungsebene mittels Bildauswertung; und

Bestimmen des Fern-Durchblickpunkts als den Schnittpunkt einer in einer horizontalen Ebene liegenden virtuellen Blickrichtung mit der Fassungsebene, wobei die virtuelle Blickrichtung als die Richtung einer virtuellen Geraden bestimmt wird, die durch Drehen einer durch einen auf dem Auge angeordneten Referenzpunkt bekannter Lage in Bezug auf die Kamera oder der Brillenfassung und den Schnittpunkt der Blickrichtung festgelegten Geraden ermittelt wird, indem diese Gerade zum einen in einem von dem Referenzunkt beabstandeten, innerhalb des Auges auf dieser Geraden liegenden virtuellen Drehpunkt um einen von dem korrigierten Kopfdrehwinkel ($\beta$') abhängigen ersten Winkel ($\beta_{OD}$", $\beta_{OS}$") um eine zu der vertikalen Richtung parallele Achse gedreht wird und indem diese Gerade zum anderen in dem virtuellen Drehpunkt um eine zu der Abstandslinie der Pupillen der Augen des Probanden parallele weitere Achse um einen von dem korrigierten Vorneigungswinkel ($\alpha$) abhängigen weiteren Winkel ($\alpha$") gedreht wird.

Für den von dem korrigierten Kopfdrehwinkel ($\beta$') abhängigen ersten Winkel ($\beta_{OD}$") gilt, wenn das Auge 60 ein rechtes Auge des Probanden 68 ist: tan $\beta_{OD}$" = (0,5 $\times$ $P_D$ $\times$ cos $\beta$' + YZ) / (D + HSA).

Für den von dem korrigierten Kopfdrehwinkel ($\beta$') abhängigen ersten Winkel ($\beta_{OS}$") gilt, wenn das Auge 62 ein linkes Auge des Probanden 68 ist: tan $\beta_{OS}$" = (0,5 $\times$ $P_D$ $\times$ cos $\beta$' - YZ) / (D + HSA).

$P_D$ ist dabei die Pupillendistanz der Augen des Probanden, D + HSA der senkrechte Abstand der Kamera von einer vertikalen Ebene, in der sich die Abstandslinie der Pupillen der Augen befindet, und YZ die Ablage des Schnittpunkts (Y) der Abstandslinie der Pupillen der Augen mit der senkrechten Projektion der optischen Achse der Kamera in eine horizontale Ebene, in der sich die Abstandlinie der Pupillen der Augen befindet, von dem Schnittpunk (Z) der Abstandslinie der Pupillen der Augen mit einer die Abstandslinie der Pupillen der Augen schneidenden vertikalen und zu der Fassungsebene senkrechten Ebene, in der die vertikale Symmetrieachse der Brillenfassung liegt.

Für den von dem korrigierten Vorneigungswinkel ($\alpha$) abhängigen weiteren Winkel ($\alpha$") gilt: $\alpha$" = - ($\gamma$ + $\delta$), wobei $\delta$ der Winkel ist, den die optische Achse der Kamera mit der senkrechten Projektion der Blickrichtung in eine vertikale Ebene bildet, in der die optische Achse der Kamera liegt.

Ein erfindungsgemäßes Computerprogramm kann auch Programmcode für das Analysieren einer Aufnahme des Probanden von der Seite mit wenigstens drei ein weiteres Koordinatensystem definierenden, zu der Brillenfassung ortsfesten Seitenzielmarken mittels Bildauswertung für das Bestimmen des Hornhautscheitelabstand (HSA) enthalten.

Im Folgenden wird die Erfindung anhand eines in der Zeichnung in schematischer Weise dargestellten Ausführungsbeispiels näher erläutert.

Es zeigen:

Fig. 1      ein System zum Bestimmen von Anpassparametern für ein in einer Brillenfassung mit einem Messbügel aufgenommenes Brillenglas und mit einer Kamera in einer Ansicht von oben;

Fig. 2      das System zum Bestimmen von Anpassparameters in einer Seitenansicht;

Fig. 3      eine Draufsicht auf eine Brillenfassung;

Fig. 4      eine Seitenansicht einer Brillenfassung;

Fig. 5a      und Fig. 5b Ansichten von Frontzielmarken des Messbügels bei einer Brillenfassung ohne Vorneigung;

Fig. 6a      und Fig. 6b Ansichten von Frontzielmarken des Messbügels bei einer Brillenfassung mit Vorneigung; und

Fig. 7a      und Fig. 7b Ansichten von Frontzielmarken des Messbügels für eine Kopfhaltung des Probanden, bei der dieser nicht in die Kamera blickt.

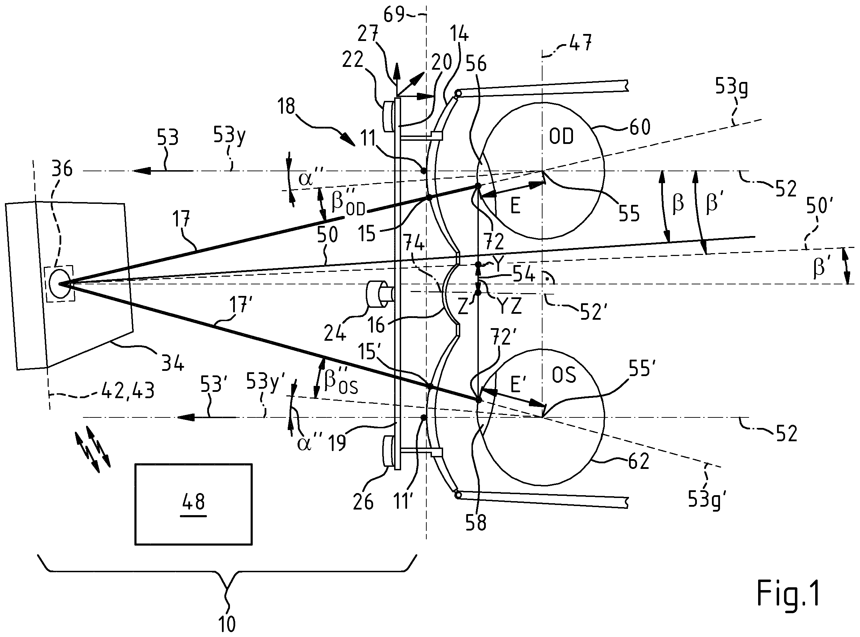

Das in der Fig. 1 gezeigte System 10 erlaubt einem Anwender das Bestimmen des in der Norm EN ISO 13666 definierten Fern-Durchblickpunkts 11, 11' und weiterer Anpassparameter für ein erstes Brillenglas 12 und ein zweites Brillenglas 14, die in eine an die Anatomie eines Brillenträgers bereits angepasste Brillenfassung 16 gefasst werden sollen. Das System 10 enthält hierzu einen Messbügel 18, der an der Brillenfassung 16 lösbar befestigt werden kann. Der Messbügel 18 kann z. B. den in der DE 10 2004 063 981 B4 beschriebenen Aufbau haben.

Für das Anpassen an unterschiedliche Fassungsgeometrien hat der Messbügel 18 eine verstellbare Traverse 20 und zwei schwenkbeweglich gelagerte verstellbare Schenkel (nicht gezeigt). Mit an der Traverse 20 und den verstellbaren Schenkeln angeordneten Fassungsaufnahmen kann der Messbügel 18 an einer Brillenfassung 16 festgeklemmt werden.

Der Messbügel 18 ist an seiner Vorderseite 19 mit einer linken, einer mittleren und einer rechten Frontzielmarke 22, 24, 26 versehen. Die Frontzielmarken 22, 24, 26 sind im Bereich der Traverse 20 angeordnet. Dabei sind die linke und rechte Frontzielmarke 22, 26 gegenüber der mittleren Frontzielmarke 24 zurückgesetzt positioniert. Die Frontzielmarken 22, 24, 26 definieren ein zu dem Messbügel 18 und zu der Brillenfassung 16, an die der Messbügel 18 angeschlossen ist, ortsfestes Koordinatensystem 27.

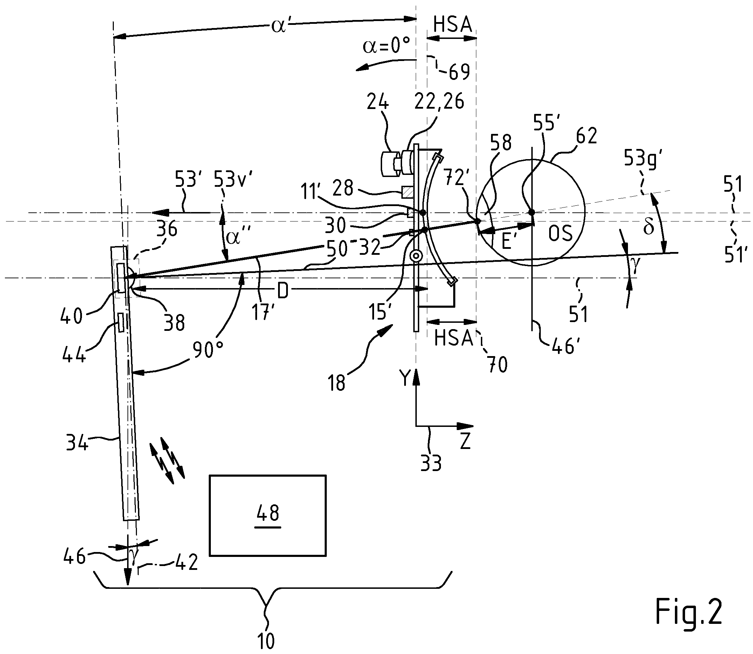

Die Fig. 2 ist eine Seitenansicht des Systems 10. Der Messbügel 18 hat an seiner linken und rechten Seite jeweils drei Seitenzielmarken 28, 30, 32, die zwischen den beiden verstellbaren Schenkeln und der Traverse 20 angeordnet sind. Die Seitenzielmarken 28, 30, 32 definieren ein weiteres Koordinatensystem 33, das zu dem Koordinatensystem 27 des Messbügels 18 und der Brillenfassung 16 referenziert ist. Wie die Fig. 2 zeigt, ist die Seitenzielmarke 28 des Messbügels 18 in der x-Richtung des Koordinatensystems 33 gegenüber den Seitenzielmarken 30, 32 zurückgesetzt positioniert.

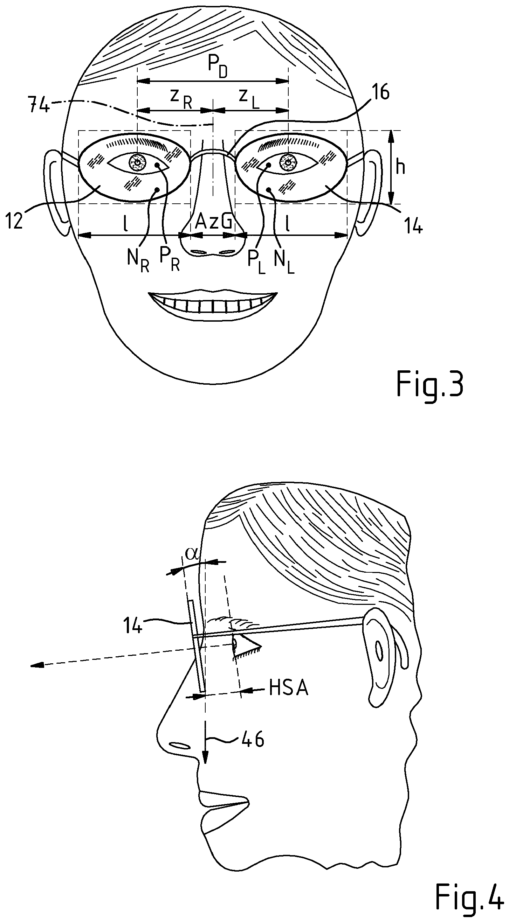

Die Fig. 3 ist eine Draufsicht auf die Fassungsebene und zeigt darin die vertikale Symmetrieachse 74 der Brillenfassung 16 sowie unterschiedliche Anpassparameter für die Brillengläser 12, 14 in der Brillenfassung 16. Die Fig. 4 zeigt eine Seitenansicht der Brillenfassung mit dem Auge eines Probanden.

Für das Bestimmen des Fern-Durchblickpunkts 11, 11' und weiterer Anpassparameter wird in dem in der Fig. 1 und Fig. 2 gezeigten System 10 ein Abschnitt der einem Proband 68 aufgesetzten Brillenfassung 16 digital erfasst. Das System 10 weist hierzu einen Tabletcomputer 34 auf, der eine Kamera 36 enthält. Der Tabletcomputer 34 hat einen berührungssensitiven Bildschirm 38. Die Kamera 36 hat eine Abbildungsoptik 38 und enthält einen Bildsensor 40, der in einer Kamera-Bildebene 42 angeordnet ist.

Der Tabletcomputer 34 enthält einen Neigungssensor 44, mit dem der Winkel $\gamma$ der Neigung der Kamera-Bildebene 42 gegenüber der Richtung 46 der Schwerkraft erfasst werden kann. Der Neigungssensor 44 ermöglicht es, die Neigung der Kamera-Bildebene 42 gegenüber der vertikalen Richtung 46 der Schwerkraft zu erfassen, d. h. er ermöglicht die Neigung der Kamera-Bildebene 42 um eine zu der Kamera-Bildebene 42 parallele horizontale Achse 43 zu bestimmen, wenn mit der Kamera 36 in dem Abschnitt der einem Proband aufgesetzten Brillenfassung 16 ein Bild der einem Proband 68 aufgesetzten Brillenfassung 16 aufgenommen wird. Für das Erfassen von Bildern mit dem dazugehörenden Winkel $\gamma$ der Neigung der Kamera-Bildebene 42 enthält der Tabletcomputer 34 ein Anwendungsprogramm (App).

Das System 10 hat eine als Servercomputer ausgebildete Rechnereinheit 48. Die Rechnereinheit 48 ist mit dem Tabletcomputer 34 mittels der WLAN-Übertragungstechnik drahtlos verbunden. Zu einem mit der Kamera 36 in dem Tabletcomputer 34 aufgenommenen Bild erhält die Rechnereinheit 48 die mittels des Bildsensors 40 erfassten digitalen Bilddaten und den Winkel $\gamma$ der Neigung der Kamera-Bildebene 42 gegenüber der Richtung 46 der Schwerkraft.

Für die Anpassparameterbestimmung enthält die Rechnereinheit 48 ein Computerprogramm. Das Computerprogramm weist einen Rechenalgorithmus auf, mittels dessen aus einem Bild des Abschnitts der dem Proband aufgesetzten Brillenfassung 16 mit dem daran angeschlossenen Messbügel 18, das die drei an dessen Traverse 20 angeordnete Frontzielmarken 22, 24, 26 enthält, durch digitale Bildanalyse, d. h. durch Bildauswertung den auf die Richtung 46 der Schwerkraft bezogenen Vorneigungswinkel $\alpha$ der Brillenfassung 16 sowie den Kopfdrehwinkel $\beta$ in Bezug auf die optische Achse 50 der Kamera 36 ermittelt. Der Kopfdrehwinkel $\beta$ ist also derjenige Winkel, den die optische Achse 50 der Kamera 36 mit einer Ebene 52 bildet, die zu der Abstandslinie 54 der Pupillen 56, 58 der Augen 60, 62 des Probanden 68, d. h. zu einer gedachten Verbindungslinie der Augen 60, 62 des Probanden 68 senkrecht ist.

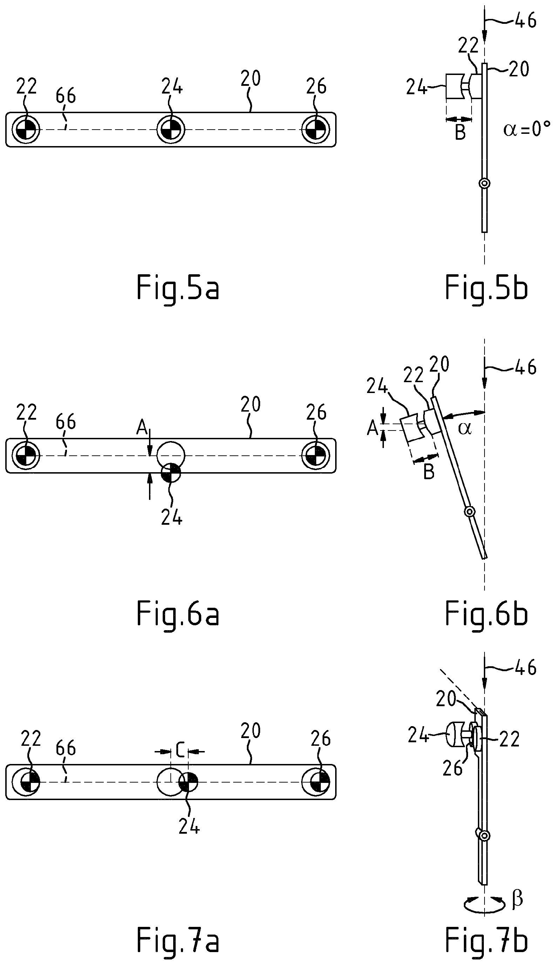

Der Vorneigungswinkel $\alpha$ der Brillenfassung 16 und der Kopfdrehwinkel $\beta$ wird dabei aus der Lage der Frontzielmarken 22, 24, 26 in der Bildebene 42 der Kamera 36 berechnet. Hier wird ausgenützt, dass die mittlere Frontzielmarke 24, wie die Fig. 5a und die Fig. 5b zeigt, von der Ebene, in der die Frontzielmarken 22 und 26 des Messbügels 18 liegen, den Abstand B hat.

Damit wird erreicht, dass wenn die Brillenfassung 16 mit dem Messbügel 18 einen Vorneigungswinkel $\alpha=0°$ gegenüber der vertikale Richtung 46 aufweist und wenn für den Neigungswinkel $\gamma$ der Bildebene 42 der Kamera 36 gegenüber der vertikalen Richtung 46 ebenfalls gilt: $\gamma=0$, die Kamera 36 die Frontzielmarken 22, 24, 26, wie sich aus der Fig. 5a ergibt, als ein Bild erfasst, in dem diese auf einer gedachten Verbindungslinie 66 liegen. Ist aber die Brillenfassung 16 dagegen, wie in der Fig. 6b gezeigt, gegenüber der vertikalen Richtung 46 geneigt, so ist das mittels der Kamera 36 erfasste Bild der mittleren Frontzielmarke 24 in der Bildebene 42 der Kamera 36, wie in der Fig. 6a zu sehen ist, gegenüber einer gedachten Verbindungslinie 66 zwischen dem Bild der Frontzielmarke 22 und dem Bild der Frontzielmarke 26 um einen Betrag A verschoben. Aus dem bekannten Abstand B der Frontzielmarke 24 von der Verbindungslinie der Frontzielmarken 22, 26 kann deshalb der gesuchte Vorneigungswinkel $\alpha$ zu $\alpha = \arctan(A/B)$ ermittelt werden.

Ist die Bildebene 42 der Kamera 36 um eine zu der Bildebene 42 parallele horizontale Achse entsprechend dem Winkel $\gamma$ geneigt, so korrigiert das Computerprogramm einen auf der Grundlage der vorstehend beschriebenen Beziehung ermittelten Vorneigungswinkel $\alpha'$ der Brillenfassung 16 zu der Bildebene 42 der Kamera noch um den mittels des Neigungssensors 44 erfassten Winkel $\gamma$ zu einem auf die vertikale Richtung 46 bezogenen Vorneigungswinkel a.

Wenn die optische Achse 50 der Kamera 36 mit der Ebene 52 den Kopfdrehwinkel $\beta > 0$ einschließt, so ist das mittels der Kamera 36 erfasste Bild der mittleren Frontzielmarke 24 in der Bildebene 42 der Kamera 36, wie in der Fig. 7a und der Fig. 7b zu sehen ist, in Bezug auf die Ansicht der Fig. 7a zwischen dem Bild der Frontzielmarke 22 und dem Bild der Frontzielmarke 26 ebenfalls um einen Betrag C verschoben. Für den auf die vertikale Richtung 46 bezogenen Kopfdrehwinkel $\beta'$ gilt hier: $\beta' = \arctan(C/B(\cos(\alpha'-\gamma))$.

Aus der mittels Bildauswertung erfassten Relativposition der Frontzielmarken 22, 24 und 26 in der Bildebene 42 der Kamera 36 errechnet das Computerprogramm in der Rechnereinheit 48 dann auf dieser Grundlage den Kopfdrehwinkel $\beta$.

Ist die Bildebene 42 der Kamera 36 um eine zu der Bildebene 42 parallele horizontale Achse entsprechend dem Winkel γ geneigt, so korrigiert das Computerprogramm den auf der Grundlage der vorstehend beschriebenen Beziehung ermittelten Kopfdrehwinkel β entsprechend dem mittels des Neigungssensors 44 erfassten Winkel γ zu einem korrigierten Kopfdrehwinkel β', der einer horizontalen Ausrichtung der optischen Achse 50 der Kamera entspricht. Der korrigierte Kopfdrehwinkel β' ist also der Winkel, den die in der Fig. 1 gezeigte senkrechte Projektion 50' der optischen Achse 50 der Kamera 36 in eine horizontale Ebene mit der zu der Abstandlinie 54 der Pupillen 56, 58 der Augen 60, 62 des Probanden 68 senkrechten, im Allgemeinen vertikalen Ebene 52 bildet.

Mittels des Computerprogramms wird dann das Bild des Abschnitts der dem Proband 68 aufgesetzten Brillenfassung 16 mit dem daran angeschlossenen Messbügel 18, das die drei an dessen Traverse 20 angeordnete Frontzielmarken 22, 24, 26 enthält, in einen Bilddatensatz umgerechnet, der entsprechend dem Winkel γ der Neigung der Bildebene 42 der Kamera 36 um die horizontale Achse 43 und dem korrigierten, einer horizontalen Ausrichtung der optischen Achse 50 der Kamera entsprechenden Kopfdrehwinkel β' korrigiert ist, so dass dieser Bilddatensatz dann einem Kamerabild entspricht, bei dem der Proband in die Kamera 36 blickt und die Bildebene 42 der Kamera 36 exakt vertikal ausgerichtet ist. Bei dieser Umrechnung berücksichtigt das Computerprogramm die unterschiedliche Entfernung des Messbügels 18, der Brillenfassung 16 und der Pupillen 56, 58 der Augen 60, 62 des Probanden von der Kamera 36 wie folgt:

Das Computerprogramm bestimmt mittels Bildauswertung den in der Fig. 1 gezeigten Schnittpunkt 15 bzw. 15' der Blickrichtung 17 bzw. 17' des rechten und linken Auges 60 bzw. 62 des Probanden 68 mit der Fassungsebene 69 der Brillenfassung 16. Der Fern-Durchblickpunkt 11 bzw. 11' wird als der Schnittpunkt einer in einer horizontalen Ebene 51 liegenden virtuellen Blickrichtung 53, 53' mit der Fassungsebene 69 bestimmt.

Dabei wird die virtuelle Blickrichtung 53 bzw. 53' als die Richtung einer virtuellen Geraden 53v bzw. 53v' bestimmt, die aus einer Geraden 53g bzw. 53g' durch Drehen um einen virtuellen Drehpunkt 55 bzw. 55' hervorgeht. Die Gerade 53g bzw. 53g' entspricht der Blickrichtung des rechten bzw. linken Auges 60, 62 des Probanden. Die Gerade 53g bzw. 53g' ist durch einen auf dem Auge 60, 62 angeordneten Referenzpunkt 72 bzw. 72' bekannter Lage in Bezug auf die Kamera 36 oder der Brillenfassung 16 und den Schnittpunkt 15 bzw. 15' der Blickrichtung 17 bzw. 17' festgelegt. Um eine Gerade 53g bzw. 53g' in die virtuelle Gerade 53v bzw. 53v' zu überführen, wird die Gerade 53g bzw. 53g' zum einen in einem von dem Referenzunkt 72 bzw. 72' beabstandeten, innerhalb des Auges 60, 62 auf der Geraden 53g bzw. 53'g liegenden virtuellen Drehpunkt 55, 55' um einen von dem korrigierten Kopfdrehwinkel β' abhängigen ersten Winkel $\beta_{OD}$" bzw. $\beta_{OS}$" um eine zu der vertikalen Richtung 46 parallele Achse 46' gedreht. Der virtuelle Drehpunkt 55, bzw. 55' liegt auf der Geraden 53g bzw. 53g' und hat von dem Referenzpunkt 72 bzw. 72' den vorgegebenen, in der Fig. 1 ersichtlichen Abstand E bzw. E'. Für den Abstand E und den Abstand E' gilt z. B. E = E' = 15,5 mm oder E=E'=12,5 mm.

Zum anderen wird die Gerade 53g bzw. 53g' in dem virtuellen Drehpunkt 55 bzw. 55' um eine zu der Abstandslinie der Pupillen der Augen des Probanden parallele weitere Achse um einen von dem korrigierten Vorneigungswinkel (α) abhängigen weiteren Winkel (α") gedreht. Für den von dem korrigierten Kopfdrehwinkel (β') abhängigen ersten Winkel ($\beta_{OD}$") gilt dabei, wenn das Auge 60 ein rechtes Auge des Probanden 68 ist: tan $\beta_{OD}$" = (0,5 × $P_D$ × cos β' + YZ) / (D + HSA). Wenn das Auge 62 ein linkes Auge des Probanden 68 ist, gilt für den von dem korrigierten Kopfdrehwinkel (β') abhängigen ersten Winkel ($\beta_{OS}$"): tan $\beta_{OS}$" = (0,5 × $P_D$ × cos β' - YZ) / (D + HSA). $P_D$ ist die Pupillendistanz der Augen des Probanden 68 und D + HSA ist der senkrechte Abstand der Kamera 36 von der vertikalen Ebene 70, in der sich die Abstandslinie 54 der Pupillen der Augen 60 bzw. 62 befindet. YZ ist die Ablage des Schnittpunkts (Y) der Abstandslinie 54 der Pupillen 56, 58 der Augen 60, 62 mit der senkrechten Projektion 50' der optischen Achse 50 der Kamera 36 in eine horizontale Ebene 51', in der sich die Abstandlinie 54 der Pupillen 56, 58 der Augen 60, 62 befindet, von dem Schnittpunk (Z) der Abstandslinie 54 der Pupillen 56, 58 der Augen 60, 62 mit einer die Abstandslinie 54 der Pupillen 56, 58 der Augen 60, 62 schneidenden vertikalen und zu der Fassungsebene 69 senkrechten Ebene 52', in der die vertikale Symmetrieachse 74 der Brillenfassung 16 liegt. Für den von dem korrigierten Vorneigungswinkel (α) abhängigen weiteren Winkel (α") gilt dabei: α" = - (γ + δ). δ ist der Winkel, den die optische Achse 50 der Kamera 36 mit der senkrechten Projektion der Blickrichtung 17, 17' in eine vertikale Ebene bildet, in der die optische Achse 50 der Kamera 36 liegt.

Für das Bestimmen des Fern-Durchblickpunkts berücksichtigt das Computerprogramm bevorzugt die Lage des Messbügels 18 in Bezug auf die in der Fig. 1 und Fig. 2 gezeigte Fassungsebene 69 der Brillenfassung 16, die, wie in der DE 10 2004 063 981 B4 beschrieben, konstruktiv vorgegeben und damit bekannt ist. Das Computerprogramm berücksichtigt darüber hinaus bevorzugt einen gemäß der Norm EN ISO 13666:2012 definierten Hornhautscheitelabstand HSA als eine bekannte Lage eines zu dem Proband 68 ortsfesten Referenzpunkts 72, 72' in dem mittels der drei zu der Brillenfassung 16 ortsfesten Frontzielmarken 22, 24, 26 definierten Koordinatensystem 27 oder in einem zu der Brillenfassung 16 oder der Kamera 36 referenzierten Koordinatensystem (nicht gezeigt). Der Hornhautscheitelabstand HSA kann hierfür z. B. aus einer Datenbank ausgelesen und dem Computerprogramm zugeführt werden. Er kann insbesondere einem Durchschnittswert unterschiedlicher Probanden 68 entsprechen, z. B. einem Durchschnittswert einer bestimmten Population von Probanden oder bestimmter Subgruppen einer Population von Probanden. Alternativ hierzu kann auch vorgesehen sein, dass das Computerprogramm den in einer vorausgehenden Messung mittels eines PD-Messstabes oder durch Analysieren einer Aufnahme des Probanden 68 mit dem an der Brillenfassung 16 festgelegten Messbügel 18 von der Seite mittels Bildauswertung bestimmen Hornhautscheitelabstand (HSA) erhält. Damit berücksichtigt das Computerprogramm für das Bestimmen des Fern-Durchblickpunkts 11, 11' Parallaxefehler in dem mittels der Kamera 36 erfassten Bild des Probanden 68, deren Ursache ein endlicher Abstand der Augen 60, 62 des Probanden 68 von der Fassungsebene 69 der Brillenfassung 16 ist. Damit wird erreicht, dass aus dem Bild auf dem Messbügel 18 und der Brillenfassung 16 angeordneter Punkte in der Bildebene 42 der Kamera 36 und aus dem Bild auf dem Probanden 68 angeordneter Referenzpunkte 72, 72' in der Bildebene 42 der Kamera 36 auf die genaue Kopfhaltung des Probanden 68 gegenüber der Kamera 36 geschlossen werden kann.

Zu bemerken ist, dass der Referenzpunkt 72, 72' auch als eine Mitte der Pupille des Auges 60, 62 des Probanden 68 definiert sein kann, die mittels Bildauswertung ermittelt oder die von einem Augenoptiker individuell in einem Bild festgelegt wird, das die Augen 60, 62 des Probanden 68 enthält.

Das Bestimmen von Anpassparametern erfolgt dann aus dem Fern-Durchblickpunkt und aus dem erfassten Bild mittels Bildauswertung.

Für das Bestimmen von Anpassparametern bei einem Probanden 68 hat ein Anwender des Systems 10 eine große Freiheit bei dem Erfassen von Bildern des Abschnitts der dem Proband 68 aufgesetzten Brillenfassung 16 mit einem an die Brillenfassung 16 montierten Messbügel 18. Es ist nämlich bei dem erfindungsgemäßen Verfahren zum Bestimmen wenigstens eines Anpassparameters für ein in einer Brillenfassung aufnehmbares Brillenglas nicht erforderlich, dass die Richtung der optischen Achse 50 der Abbildungsoptik 38 der Kamera 36 des Tabletcomputers 34 zu dem Kopf des Probanden 68 weist. Die Erfinder konnten insbesondere zeigen, dass die Genauigkeit für das Erfassen von Brillenglas-Anpassparametern in dem System 10 auch dann nicht beeinträchtigt wird, wenn für den Neigungswinkel $\gamma$ zur vertikalen Richtung 46 der Bildebene 42 der Kamera 36 in dem Tablet-computer 34 gilt: -20° $\leq \gamma \leq$ 20°.

Zu bemerken ist, dass bei einer alternativen Ausführungsform der Erfindung vorgesehen sein kann, den Vorneigungswinkel $\alpha$' der Brillenfassung 16 zu der Bildebene 42 der Kamera 36 und den Kopfdrehwinkel $\beta$, ohne dass an der Brillenfassung 16 ein Messbügel 18 mit Frontzielmarken 22, 24, 26 angebracht ist, allein mittels Bildauswertung eines Bilds der Brillenfassung 16 zu ermitteln, indem bei der Bildauswertung eine bekannte Geometrie der Brillenfassung 16 berücksichtigt wird.

In dem System 10 können als Anpassparameter auch die Durchblickpunkte für Ferne unter Berücksichtigung des Neigungswinkels $\gamma$ bestimmt werden. Aus dem Punkt, durch den in einem erfassten Bild der Proband auf die Kamera blickt, lässt sich der Fern-Durchblickpunkt $P_{R/L}$ (rechter/linker Zentrierpunkt) bestimmen. Ebenso kann ein Nahdurchblickpunkt $N_{R/L}$ (rechter/linker Nah-Durchblickpunkt) bestimmt werden, der zusammen mit dem Fern-Durchblickpunkt $P_{R/L}$ ein Gleitsichtglas definiert. Neben den Nahdurchblickpunkten $N_{R/L}$ lässt sich auch der Winkel $\epsilon$ bestimmen zwischen der Blickrichtung des Auges 60, 62 eines Probanden 68 beim Blick in die Ferne und der Blickrichtung beim Blick in die Nähe, z. B. beim Lesen. Bei dem System 10 ist es für eine Anpassparameterbestimmung insbesondere nicht erforderlich, dass eine Lesesituation mit einer zusätzlichen, weiteren Kamera oder zusammen mit einer zusätzlichen weiteren Kamera erfasst und anschließend ausgewertet wird.

Weil das Computerprogramm der Rechnereinheit 48 in dem System 10 wie vorstehend beschrieben den gemäß der Norm EN ISO 13666:2012 definierten Hornhautscheitelabstand HSA als eine bekannte Lage eines zu dem Proband 68 ortsfesten Referenzpunkts 72, 72' in dem mittels der drei zu der Brillenfassung 16 ortsfesten Frontzielmarken 22, 24, 26 definierten Koordinatensystem 27 berücksichtigt, kann beim Bestimmen der Nahdurchblickpunkte mit dem System 10 erkannt werden, wenn der Proband 68 beim Blick gerade aus in die Kamera 36 seine habituelle Kopfhaltung einnimmt und infolgedessen seinen Kopf entsprechend seinem Führungsauge leicht verdreht hält, dass die tatsächliche Position der Nahdurchblickpunkte von einer symmetrisch nasal verschobenen Position abweicht.

Der Rechenalgorithmus des Computerprogramms in der Rechnereinheit 48 des Systems 10 ist so ausgelegt, dass damit nicht nur der Vorneigungswinkel $\alpha$ der Brillenfassung (Fassungsvorneigung), sondern alternativ oder zusätzlich auch das Fassungsmaß (I, h, AzG), die Pupillendistanz (PD, $z_R$, $z_L$), der Zentrierpunktabstand ($x_R$, $y_R$, $x_L$, $y_L$), der Fassungsscheibenwinkel und der erforderlicher Rohglasdurchmesser bestimmbar ist. Aus einer Aufnahme des Probanden 68 von der Seite, wenn dieser die Brillenfassung 16 mit dem daran festgelegten Messbügel 18 trägt, kann das Computerprogramm in der Rechnereinheit 48 den Hornhautscheitelabstand (HSA) bestimmen.

Zusammenfassend sind insbesondere folgende Merkmale der Erfindung festzuhalten: Bei einem Verfahren zum Bestimmen eines Fern-Durchblickpunkts auf einem in einer Brillenfassung 16 aufnehmbaren Brillenglas 12, 14, die eine Fassungsebene 69 hat, wird ein in einer Bildebene 42 liegendes Bild wenigstens eines Abschnitts einer einem Proband 68 aufgesetzten Brillenfassung 16 mit einer eine optische Achse 50 aufweisenden Kamera 36 erfasst, das die Pupillen 56, 58 der Augen 60, 62 des Probanden 68 enthält, während der Proband 68 mit einer die Fassungsebene 69 durchsetzenden Blickrichtung 17, 17' wenigstens eines Auges 60, 62 in die Kamera 36 blickt. Dabei wird ein auf die Lage der Bildebene 42 bezogener Vorneigungswinkel $\alpha$' der Brillenfassung 16 ermittelt. Der Vorneigungswinkel $\alpha$' wird entsprechend dem erfassten Neigungswinkel $\gamma$ der Bildebene 42 gegenüber der vertikalen Richtung 46 zu einem auf die vertikale Richtung 46 bezogenen Vorneigungswinkel $\alpha$ korrigiert. Es wird ein von der optischen Achse 50 der Kamera 36 mit einer zu der Abstandslinie 54 der Pupillen 56, 58 der Augen 60, 62 des Probanden 68 senkrechten Ebene gebildeter Kopfdrehwinkel $\beta$ des Kopfs des Probanden 68 ermittelt und der Kopfdrehwinkel $\beta$ entsprechend dem erfassten

Neigungswinkel γ der Bildebene 42 gegenüber der vertikalen Richtung 46 zu einem einer horizontalen Ausrichtung der optischen Achse 50 der Kamera 36 entsprechenden korrigierten Kopfdrehwinkel korrigiert. Der Schnittpunkt 15' der Blickrichtung 17, 17' mit der Fassungsebene 69 wird dann mittels Bildauswertung bestimmt und daraus der Fern-Durchblickpunkt 11, 11' als der Schnittpunkt einer in einer horizontalen Ebene 51 liegenden virtuellen Blickrichtung 53, 53' mit der Fassungsebene 69. Die virtuelle Blickrichtung 53, 53' wird als die Richtung einer virtuellen Geraden 53v, 53v' bestimmt, die durch Drehen einer durch einen auf dem Auge 60, 62 angeordneten Referenzpunkt 72, 72' bekannter Lage in Bezug auf die Kamera 36 oder die Brillenfassung 16 und den Schnittpunkt 15, 15' der Blickrichtung 17, 17' festgelegten Geraden 53g, 53'g ermittelt wird. Hierzu wird diese Gerade 53g, 53'g zum einen in einem von dem Referenzunkt 72, 72' beabstandeten, innerhalb des Auges 60, 62 auf dieser Geraden 53g, 53g' liegenden virtuellen Drehpunkt 55, 55' um einen von dem korrigierten Kopfdrehwinkel (β') abhängigen ersten Winkel $\beta_{OD}$", $\beta_{OS}$" um eine zu der vertikalen Richtung 46 parallele Achse 46' gedreht. Zum anderen wird diese Gerade 53g, 53g' in dem virtuellen Drehpunkt 55, 55' um eine zu der Abstandslinie 54 der Pupillen 56, 58 der Augen 60, 62 des Probanden 68 parallele weitere Achse 47 um einen von dem korrigierten Vorneigungswinkel (α) abhängigen weiteren Winkel (α") gedreht. Für den von dem korrigierten Kopfdrehwinkel (β') abhängigen ersten Winkel $\beta_{OD}$" gilt, wenn das Auge 60 ein rechtes Auge des Probanden 68 ist: tan $\beta_{OD}$" = (0,5 × $P_D$ × cos β' + YZ) / (D + HSA). Für den von dem korrigierten Kopfdrehwinkel (β') abhängigen ersten Winkel $\beta_{OS}$" gilt, wenn das Auge 62 ein linkes Auge des Probanden 68 ist: tan $\beta_{OS}$" = (0,5 × $P_D$ × cos β' - YZ) / (D + HSA). $P_D$ ist dabei die Pupillendistanz der Augen des Probanden 68. D + HSA ist der senkrechte Abstand der Kamera 36 von einer vertikalen Ebene 70, in der sich die Abstandslinie 54 der Pupillen der Augen 60, 62 befindet. YZ ist die Ablage des Schnittpunkts (Y) der Abstandslinie 54 der Pupillen 56, 58 der Augen 60, 62 mit der senkrechten Projektion 50' der optischen Achse 50 der Kamera 36 in eine horizontale Ebene 51', in der sich die Abstandlinie 54 der Pupillen 56, 58 der Augen 60, 62 befindet, von dem Schnittpunk (Z) der Abstandslinie 54 der Pupillen 56, 58 der Augen 60, 62 mit einer die Abstandslinie 54 der Pupillen 56, 58 der Augen 60, 62 schneidenden vertikalen und zu der Fassungsebene 69 senkrechten Ebene 52', in der die vertikale Symmetrieachse 74 der Brillenfassung 16 liegt. Für den von dem korrigierten Vorneigungswinkel (α) abhängigen weiteren Winkel a" gilt: α" = - (γ + δ), wobei δ der Winkel ist, den die optische Achse 50 der Kamera 36 mit der senkrechten Projektion der Blickrichtung 17, 17' in eine vertikale Ebene bildet, in der die optische Achse 50 der Kamera 36 liegt.

**Bezugszeichenliste:**

| | |
|---|---|
| 10 | System |
| 11, 11' | Fern-Durchblickpunkt |
| 12, 14 | Brillenglas |
| 15, 15' | Schnittpunkt |
| 16 | Brillenfassung |
| 17, 17' | Blickrichtung |
| 18 | Messbügel |
| 19 | Vorderseite |
| 20 | Traverse |
| 22, 24, 26 | Frontzielmarke |
| 27 | Koordinatensystem |
| 28, 30, 32 | Seitenzielmarken |
| 33 | Koordinatensystem |
| 34 | Tabletcomputer |
| 36 | Kamera |
| 38 | Abbildungsoptik / Bildschirm |
| 40 | Bildsensor |
| 42 | Bildebene |
| 43 | horizontale Achse |
| 44 | Neigungssensor |
| 46 | vertikale Richtung |
| 46' | zu der vertikalen Richtung parallele Achse |
| 47 | zu der Achse 46' und der Ebene 52 senkrechte Achse |
| 48 | Rechnereinheit |
| 50 | optische Achse |
| 50' | Projektion der optischen Achse |
| 51, 51' | horizontale Ebene |

| 52 | vertikale Ebene |
| 52' | vertikale Ebene durch Symmetrieachse der Brillenfassung |
| 53, 53' | virtuelle Blickrichtung |
| 53g, 53g' | Gerade |
| 53v, 53v' | virtuelle Gerade durch virtuellen Drehpunkt eines Auges |
| 54 | Abstandslinie der Pupillen |
| 55, 55' | virtueller Drehpunkt |
| 56, 58 | Pupillen |
| 60, 62 | Augen |
| 66 | Verbindungslinie |
| 68 | Proband |
| 69 | Fassungsebene |
| 70 | vertikale Ebene |
| 72, 72' | Referenzpunkt |
| 74 | vertikale Symmetrieachse der Brillenfassung |

| $\alpha$ | auf vertikale Richtung bezogener Vorneigungswinkel |
| $\alpha'$ | auf Kamera-Bildebene bezogener Vorneigungswinkel |
| $\alpha''$ | Drehwinkel |
| $\beta$ | Kopfdrehwinkel |
| $\beta'$ | korrigierter Kopfdrehwinkel |
| $\beta_{OS}''$ | Drehwinkel |
| $\gamma$ | Neigungswinkel der Kamera-Bildebene zur Vertikalen |

| A, B, C, D | Abstand |
| HSA | Hornhautscheitelabstand |
| E, E' | Abstand virtueller Drehpunkt von Referenzpunkt auf linkem bzw. rechtem Auge |
| Y, Z | Punkt |
| YZ | Abstand des Punkts Y von dem Punkt Z |

**Patentansprüche**

1. Verfahren zum Bestimmen eines Fern-Durchblickpunkts (11, 11') auf einem in einer Brillenfassung (16) aufnehmbaren Brillenglas (12, 14), die eine Fassungsebene (69) hat,
bei dem ein in einer Bildebene (42) liegendes Bild wenigstens eines Abschnitts einer einem Proband (68) aufgesetzten Brillenfassung (16) mit einer eine optische Achse (50) aufweisenden Kamera (36) erfasst wird, das die Pupillen (56, 58) der Augen (60, 62) des Probanden (68) enthält, während der Proband (68) mit einer die Fassungsebene (69) durchsetzenden Blickrichtung (17, 17') wenigstens eines Auges (60, 62) in die Kamera (36) blickt,
bei dem ein auf die Lage der Bildebene (42) bezogener Vorneigungswinkel $\alpha'$ der Brillenfassung (16) ermittelt wird, der entsprechend dem erfassten Neigungswinkel $\gamma$ der Bildebene (42) gegenüber der vertikalen Richtung (46) zu einem auf die vertikale Richtung (46) bezogenen Vorneigungswinkel $\alpha$ korrigiert wird,
bei dem ein von der optischen Achse (50) der Kamera (36) mit einer zu der Abstandslinie (54) der Pupillen (56, 58) der Augen (60, 62) des Probanden (68) senkrechten Ebene (52) gebildeter Kopfdrehwinkel $\beta$ des Kopfs des Probanden (68) ermittelt wird,
bei dem der Kopfdrehwinkel $\beta$ entsprechend dem erfassten Neigungswinkel $\gamma$ der Bildebene (42) gegenüber der vertikalen Richtung (46) zu einem einer horizontalen Ausrichtung der optischen Achse (50) der Kamera (36) entsprechenden korrigierten Kopfdrehwinkel $\beta'$ korrigiert wird, und
bei dem der Fern-Durchblickpunkt (11, 11') durch Analysieren des in der Bildebene (42) liegenden Bilds unter Berücksichtigung des einer horizontalen Ausrichtung der optischen Achse (50) der Kamera (36) entsprechenden korrigierten Kopfdrehwinkels $\beta'$ bestimmt wird, und
bei dem der Schnittpunkt (15, 15') der Blickrichtung (17, 17') mit der Fassungsebene (69) mittels Bildauswertung bestimmt wird;
**dadurch gekennzeichnet, dass**
der Fern-Durchblickpunkt (11, 11') als der Schnittpunkt einer in einer horizontalen Ebene (51) liegenden virtuellen Blickrichtung (53, 53') mit der Fassungsebene (69) bestimmt wird, wobei
die virtuelle Blickrichtung (53, 53') als die Richtung einer virtuellen Geraden (53v, 53v') bestimmt wird, die durch Drehen einer durch einen auf dem Auge (60, 62) angeordneten Referenzpunkt (72, 72') bekannter Lage in Bezug

12

auf die Kamera (36) oder der Brillenfassung (16) und den Schnittpunkt (15, 15') der Blickrichtung (17, 17') festgelegten Geraden (53g, 53g') ermittelt wird,

indem diese Gerade (53g, 53'g) zum einen in einem von dem Referenzpunkt (72, 72') beabstandeten, innerhalb des Auges (60, 62) auf dieser Geraden (53g, 53'g) liegenden virtuellen Drehpunkt (55, 55') um einen von dem korrigierten Kopfdrehwinkel $\beta$' abhängigen ersten Winkel $\beta_{OD}$", $\beta_{OS}$" um eine zu der vertikalen Richtung (46) parallele Achse (46') gedreht wird, und

indem diese Gerade (53g, 53g') zum anderen in dem virtuellen Drehpunkt (55, 55') um eine zu der Abstandslinie (54) der Pupillen (56, 58) der Augen (60, 62) des Probanden (68) parallele weitere Achse (47) um einen von dem korrigierten Vorneigungswinkel $\alpha$ abhängigen weiteren Winkel $\alpha$" gedreht wird,

wobei für den von dem korrigierten Kopfdrehwinkel $\beta$' abhängigen ersten Winkel $\beta_{OD}$" gilt, wenn das Auge (60) ein rechtes Auge des Probanden (68) ist: $\tan \beta_{OD}$" = $(0,5 \times P_D \times \cos \beta$' + YZ$) / (D + HSA)$,

und wobei für den von dem korrigierten Kopfdrehwinkel $\beta$' abhängigen ersten Winkel $\beta_{OS}$" gilt, wenn das Auge (62) ein linkes Auge des Probanden (68) ist: $\tan \beta_{OS}$" = $(0,5 \times P_D \times \cos \beta$' - YZ$) / (D + HSA)$,

wobei $P_D$ die Pupillendistanz der Augen des Probanden (68) ist,

wobei D + HSA der senkrechte Abstand der Kamera (36) von einer vertikalen Ebene (70) ist, in der sich die Abstandslinie (54) der Pupillen der Augen (60, 62) befindet, und

wobei YZ die Ablage des Schnittpunkts (Y) der Abstandslinie (54) der Pupillen (56, 58) der Augen (60, 62) mit der senkrechten Projektion (50') der optischen Achse (50) der Kamera (36) in eine horizontale Ebene (51'), in der sich die Abstandlinie (54) der Pupillen (56, 58) der Augen (60, 62) befindet, von dem Schnittpunkt (Z) der Abstandslinie (54) der Pupillen (56, 58) der Augen (60, 62) mit einer die Abstandslinie (54) der Pupillen (56, 58) der Augen (60, 62) schneidenden vertikalen und zu der Fassungsebene (69) senkrechten Ebene (52') ist, in der die vertikale Symmetrieachse (74) der Brillenfassung (16) liegt, und

wobei für den von dem korrigierten Vorneigungswinkel ($\alpha$) abhängigen weiteren Winkel $\alpha$" gilt: $\alpha$" = - ($\gamma$ + $\delta$),

wobei $\delta$ der Winkel ist, den die optische Achse (50) der Kamera (36) mit der senkrechten Projektion der Blickrichtung (17, 17') in eine vertikale Ebene bildet, in der die optische Achse (50) der Kamera (36) liegt.

2. Verfahren nach Anspruch 1, **dadurch gekennzeichnet, dass** der auf die Lage der Bildebene (42) bezogene Vorneigungswinkel $\alpha$' der Brillenfassung (16) mittels Bildanalyse des erfassten Bilds des wenigstens einen Abschnitts der dem Proband (68) aufgesetzten Brillenfassung (16) ermittelt wird und/oder dass der von der optischen Achse (50) der Kamera (36) mit einer zu der Abstandslinie (54) der Pupillen (56, 58) der Augen (60, 62) des Probanden (68) senkrechten Ebene gebildete Kopfdrehwinkel $\beta$ des Kopfs des Probanden (68) mittels Bildanalyse des erfassten Bilds des wenigstens einen Abschnitts der dem Proband (68) aufgesetzten Brillenfassung (16) ermittelt wird.

3. Verfahren nach Anspruch 1 oder 2, **dadurch gekennzeichnet, dass** der korrigierte Vorneigungswinkel $\alpha$ der folgenden Beziehung genügt:

$$\alpha = \alpha' - \gamma.$$

4. Verfahren nach einem der Ansprüche 1 bis 3, **dadurch gekennzeichnet, dass** der korrigierten Kopfdrehwinkel $\beta$' der folgenden Beziehung genügt: $\beta$' = $\beta$ / cos ($\alpha$' - $\gamma$).

5. Verfahren nach einem der Ansprüche 1 bis 4, **dadurch gekennzeichnet, dass** der Referenzpunkt (72, 72') der Hornhautscheitelpunkt des Auges (60, 62) des Probanden (68) ist und/oder dass der Referenzpunkt (72, 72') eine festgelegte Mitte der Pupille des Auges (60, 62) des Probanden (68) ist.

6. Verfahren nach einem der Ansprüche 1 bis 5, **dadurch gekennzeichnet, dass** die Lage des virtuellen Drehpunkts (55, 55') auf der durch den Referenzpunkt (72, 72') bekannter Lage in Bezug auf die Kamera (36) oder der Brillenfassung (16) und den Schnittpunkt (15, 15') der Blickrichtung (17, 17') festgelegten Geraden (53g, 53g') durch Vorgeben eines festen Abstands (E, E') des virtuellen Drehpunkts (55, 55') von dem Referenzpunkt (72, 72') bestimmt wird.

7. Verfahren nach einem der Ansprüche 1 bis 6, **dadurch gekennzeichnet, dass** die Pupillendistanz $P_D$ der Augen (60, 62) des Probanden (68) mittels Bildanalyse eines mit der Kamera (36) erfassten Bilds ermittelt wird, das die Pupillen der Augen (60, 62) des Probanden (68) und wenigstens einen Abschnitt der dem Proband (68) aufgesetzten Brillenfassung (16) enthält.

8.  Verfahren nach einem der Ansprüche 1 bis 7, **dadurch gekennzeichnet, dass** der Winkel $\delta$, den die optische Achse (50) der Kamera (36) mit der senkrechten Projektion der Blickrichtung (17, 17') in eine vertikale Ebene bildet, mittels Bildanalyse des erfassten Bilds des wenigstens einen Abschnitts der dem Proband (68) aufgesetzten Brillenfassung (16) ermittelt wird.

9.  Verfahren nach einem der Ansprüche 1 bis 8, **dadurch gekennzeichnet, dass** die Ablage des Schnittpunkts Y der Abstandslinie (54) der Pupillen (56, 58) der Augen (60, 62) mit der senkrechten Projektion (50') der optischen Achse (50) der Kamera (36) in eine horizontale Ebene (51'), in der sich die Abstandlinie (54) der Pupillen (56, 58) der Augen (60, 62) befindet, von dem Schnittpunk Z der Abstandslinie (54) der Pupillen (56, 58) der Augen (60, 62) mit einer die Abstandslinie (54) der Pupillen (56, 58) der Augen (60, 62) schneidenden vertikalen und zu der Fassungsebene (69) senkrechten Ebene (52'), in der die vertikale Symmetrieachse (74) der Brillenfassung (16) liegt, mittels Bildanalyse des mit der Kamera (36) erfassten Bilds ermittelt wird.

10. Verfahren zum Bestimmen wenigstens eines Anpassparameters aus der Gruppe Fassungsmaß, Zentrierpunktabstand, Pupillendistanz, Rohglasdurchmesser, Fassungsvorneigung **gekennzeichnet durch** das Bestimmen eines Fern-Durchblickpunkts (11, 11') nach einem der Ansprüche 1 bis 9 und das Bestimmen des Anpassparameters aus dem bestimmten Fern-Durchblickpunkt (11, 11') und aus zusätzlichen Informationen, die aus dem erfassten Bild mittels Bildauswertung gewonnen sind.

11. System zum Bestimmen eines Fern-Durchblickpunkts (11, 11') auf einem in einer Brillenfassung (16) aufnehmbaren Brillenglas (12, 14), die eine Fassungsebene (69) hat, umfassend
    eine Kamera (36), die eine Bildebene (42) hat, mit einem die Neigung der Bildebene (42) der Kamera (36) um eine horizontale Achse (43) erfassenden Neigungssensor (44), und
    eine Rechnereinheit (48) mit
    Mitteln für das Bestimmen eines auf die Lage der Bildebene (42) bezogenen Vorneigungswinkels $\alpha'$ der Brillenfas-sung (16),
    Mitteln für das Korrigieren des Vorneigungswinkels $\alpha'$ der Brillenfassung (16) entsprechend dem erfassten Nei-gungswinkel $\gamma$ der Bildebene (42) gegenüber der vertikalen Richtung (46) zu einem auf die vertikale Richtung (46) bezogenen Vorneigungswinkel $\alpha$,
    Mitteln für das Bestimmen eines von der optischen Achse (50) der Kamera (36) mit einer zu der Abstandslinie (54) der Pupillen (56, 58) der Augen (60, 62) des Probanden (68) senkrechten Ebene gebildeten Kopfdrehwinkels $\beta$ des Kopfs des Probanden (68),
    Mitteln für das Korrigieren des Kopfdrehwinkels $\beta$ entsprechend dem erfassten Neigungswinkel $\gamma$ der Bildebene (42) gegenüber der vertikalen Richtung (46) zu einem einer horizontalen Ausrichtung der optischen Achse (50) der Kamera (36) entsprechenden korrigierten Kopfdrehwinkel $\beta'$, und
    Mitteln für das Bestimmen eines Schnittpunkts der Blickrichtung (17, 17') des Probanden (68) mit der Fassungsebene (69),
    **dadurch gekennzeichnet, dass**
    die Rechnereinheit (48) enthält:

    Mittel für das Bestimmen des Fern-Durchblickpunkts (11, 11') als der Schnittpunkt einer in einer horizontalen Ebene liegenden virtuellen Blickrichtung (53, 53') mit der Fassungsebene (69),
    durch welche die virtuelle Blickrichtung (53, 53') als die Richtung einer virtuellen Gerade (53v, 53v') bestimmt wird, die durch Drehen einer durch einen auf dem Auge (60, 62) angeordneten Referenzpunkt (72, 72') bekannter Lage in Bezug auf die Kamera (36) oder der Brillenfassung (16) und den Schnittpunkt (15, 15') der Blickrichtung (17, 17') festgelegten Geraden (53g, 53g') ermittelt wird,
    indem diese Gerade (53g, 53g') zum einen in einem von dem Referenzpunkt (72, 72') beabstandeten, innerhalb des Auges (60, 62) auf dieser Geraden (53g, 53g') liegenden virtuellen Drehpunkt (55, 55') um einen von dem korrigierten Kopfdrehwinkel $\beta'$ abhängigen ersten Winkel $\beta_{OD}''$, $\beta_{OS}''$ um eine zu der vertikalen Richtung (46) parallele Achse (46') gedreht wird, und
    indem diese Gerade (53g, 53g') zum anderen in dem virtuellen Drehpunkt (55, 55') um eine zu der Abstandslinie (54) der Pupillen (56, 58) der Augen (60, 62) des Probanden (68) parallele weitere Achse (47) um einen von dem korrigierten Vorneigungswinkel $\alpha$ abhängigen weiteren Winkel a" gedreht wird,
    wobei für den von dem korrigierten Kopfdrehwinkel $\beta'$ abhängigen ersten Winkel $\beta_{OD}''$ gilt, wenn das Auge (60) ein rechtes Auge des Probanden (68) ist: $\tan \beta_{OD}'' = (0,5 \times P_D \times \cos \beta' + YZ) / (D + HSA)$,
    und wobei für den von dem korrigierten Kopfdrehwinkel $\beta'$ abhängigen ersten Winkel $\beta_{OS}''$ gilt, wenn das Auge (62) ein linkes Auge des Probanden (68) ist: $\tan \beta_{OS}'' = (0,5 \times P_D \times \cos \beta' - YZ) / (D + HSA)$,
    wobei $P_D$ die Pupillendistanz der Augen des Probanden (68) ist,

wobei D + HSA der senkrechte Abstand der Kamera (36) von einer vertikalen Ebene (70) ist, in der sich die Abstandslinie (54) der Pupillen der Augen (60, 62) befindet, und

wobei YZ die Ablage des Schnittpunkts (Y) der Abstandslinie (54) der Pupillen (56, 58) der Augen (60, 62) mit der senkrechten Projektion (50') der optischen Achse (50) der Kamera (36) in eine horizontale Ebene (51'), in der sich die Abstandlinie (54) der Pupillen (56, 58) der Augen (60, 62) befindet, von dem Schnittpunkt (Z) der Abstandslinie (54) der Pupillen (56, 58) der Augen (60, 62) mit einer die Abstandslinie (54) der Pupillen (56, 58) der Augen (60, 62) schneidenden vertikalen und zu der Fassungsebene (69) senkrechten Ebene (52') ist, in der die vertikale Symmetrieachse (74) der Brillenfassung (16) liegt; und

wobei für den von dem korrigierten Vorneigungswinkel $\alpha$ abhängigen weiteren Winkel $\alpha''$ gilt: $\alpha'' = - (\gamma + \delta)$, wobei $\delta$ der Winkel ist, den die optische Achse (50) der Kamera (36) mit der senkrechten Projektion der Blickrichtung (17, 17') in eine vertikale Ebene bildet, in der die optische Achse (50) der Kamera (36) liegt.

12. Einrichtung zum Bestimmen wenigstens eines Anpassparameters aus der Gruppe Fassungsmaß, Zentrierpunktabstand, Pupillendistanz, Rohglasdurchmesser, Fassungsvorneigung mit einem System nach Anspruch 11, **gekennzeichnet durch** Mittel zum Bestimmen des Anpassparameters aus dem Fern-Durchblickpunkt (11, 11') und aus dem erfassten Bild mittels Bildauswertung.

13. System nach Anspruch 11 oder Einrichtung nach Anspruch 12, **dadurch gekennzeichnet, dass** die Kamera (36) in einen Tabletcomputer (34) oder in ein Mobiltelefon integriert ist und die Rechnereinheit (48) als ein mit dem Tablet-Computer oder dem Mobiltelefon verbundener Server ausgebildet ist.

14. System nach Anspruch 13, **dadurch gekennzeichnet, dass** der Tabletcomputer (34) oder das Mobiltelefon mit dem Server drahtlos kommuniziert.

15. Computerprogramm mit Programmcode zur Durchführung aller Verfahrensschritte eines Verfahrens nach einem der Ansprüche 1 bis 10, wenn das Computerprogramm in eine Rechnereinheit geladen oder in einer Rechnereinheit ausgeführt wird.


## Claims

1. Method for determining a distance visual point (11, 11') on a spectacle lens (12, 14) which is receivable in a spectacle frame (16) and which has a frame plane (69),

in which an image, lying in an image plane (42), of at least one portion of a spectacle frame (16) worn by a subject (68) is acquired by a camera (36) with an optical axis (50), which image contains the pupils (56, 58) of the eyes (60, 62) of the subject (68), while the subject (68) looks into the camera (36) with a viewing direction (17, 17') of at least one eye (60, 62) passing through the frame plane (36),

in which a pantoscopic angle $\alpha'$ of the spectacle frame (16) related to the position of the image plane (42) is established, said pantoscopic angle being corrected in accordance with the acquired inclination angle $\gamma$ of the image plane (42) in relation to the vertical direction (46) so as to form a pantoscopic angle $\alpha$ related to the vertical direction (46),

in which a head rotation angle $\beta$ of the head of the subject (68), formed by the optical axis (50) of the camera (36) with a plane (52) perpendicular to the distance line (54) of the pupils (56, 58) of the eyes (60, 62) of the subject (68), is established, in which the head rotation angle $\beta$ is corrected in accordance with the detected inclination angle $\gamma$ of the image plane (42) in relation to the vertical direction (46) so as to form a corrected head rotation angle $\beta'$ corresponding to a horizontal alignment of the optical axis (50) of the camera (36), and

in which the distance visual point (11, 11') is determined by analyzing the image lying in the image plane (42), taking into account the corrected head rotation angle $\beta'$ corresponding to a horizontal alignment of the optical axis (50) of the camera (36), and

in which the intersection (15, 15') of the viewing direction (17, 17') with the frame plane (69) is determined by means of image evaluation;

**characterized in that**

the distance visual point (11, 11') is determined as the intersection of a virtual viewing direction (53, 53') lying in a horizontal plane (51) with the frame plane (69), wherein

the virtual viewing direction (53, 53') is determined as the direction of a virtual straight line (53v, 53v'), which is established by rotating a straight line (53g, 53g') which is set by a reference point (72, 72') arranged on the eye (60, 62) with a known position in relation to the camera (36) or the spectacle frame (16) and the intersection (15, 15') of the viewing direction (17, 17'),

by virtue of this straight line (53g, 53'g) being, firstly, rotated at a virtual pivot (55, 55'), which lies at a distance from the reference point (72, 72') and on this straight line (53g, 53'g) within the eye (60, 62), about an axis (46') lying parallel to the vertical direction (46) by a first angle $\beta_{OD}$", $\beta_{OS}$" dependent on the corrected head rotation angle $\beta$', and by virtue of this straight line (53g, 53g') being, secondly, rotated at the virtual pivot (55, 55') about a further axis (47), which is parallel to the distance line (54) of the pupils (56, 58) of the eyes (60, 62) of the subject (68), by a further angle $\alpha$" dependent on the corrected pantoscopic angle $\alpha$,

wherein, if the eye (60) is a right eye of the subject (68), the following applies for the first angle $\beta_{OD}$" which is dependent on the corrected head rotation angle $\beta$': $\tan \beta_{OD}$" = (0.5 × $P_D$ × $\cos \beta$' + YZ) / (D + HSA),

and wherein, if the eye (62) is a left eye of the subject (68), the following applies for the first angle $\beta_{OS}$" which is dependent on the corrected head rotation angle $\beta$': $\tan \beta_{OS}$" = (0.5 × $P_D$ × $\cos \beta$' - YZ) / (D + HSA),

where $P_D$ is the interpupillary distance of the eyes of the subject (68),

where D + HSA is the vertical distance of the camera (36) from a vertical plane (70), in which the distance line (54) of the pupils of the eyes (60, 62) is situated, and

where YZ is the offset of the intersection (Y) of the distance line (54) of the pupils (56, 58) of the eyes (60, 62) with the vertical projection (50') of the optical axis (50) of the camera (36) into a horizontal plane (51'), in which the distance line (54) of the pupils (56, 58) of the eyes (60, 62) is situated, from the intersection (Z) of the distance line (54) of the pupils (56, 58) of the eyes (60, 62) with a vertical plane (52') intersecting the distance line (54) of the pupils (56, 58) of the eyes (60, 62), said plane being perpendicular to the frame plane (69) and the vertical axis of symmetry (74) of the spectacle frame (16) lying in said plane, and

wherein the following applies for the further angle $\alpha$" dependent on the corrected pantoscopic angle ($\alpha$):

$$\alpha'' = -(\gamma + \delta),$$

where $\delta$ is the angle which the optical axis (50) of the camera (36) forms with the perpendicular projection of the viewing direction (17, 17') into a vertical plane, in which the optical axis (50) of the camera (36) lies.

2. Method according to Claim 1, **characterized in that** the pantoscopic angle $\alpha$' of the spectacle frame (16) related to the position of the image plane (42) is established by means of image analysis of the acquired image of the at least one portion of the spectacle frame (16) worn by the subject (68) and/or in that the head rotation angle $\beta$ of the head of the subject (68), formed by the optical axis (50) of the camera (36) with a plane perpendicular to the distance line (54) of the pupils (56, 58) of the eyes (60, 62) of the subject (68), is established by means of image analysis of the acquired image of the at least one portion of the spectacle frame (16) worn by the subject (68).

3. Method according to Claim 1 or 2, **characterized in that** the corrected pantoscopic angle $\alpha$ satisfies the following relationship:

$$\alpha = \alpha' - \gamma.$$

4. Method according to any one of Claims 1 to 3, **characterized in that** the corrected head rotation angle $\beta$' satisfies the following relationship: $\beta$' = $\beta$ / $\cos$ ($\alpha$' - $\gamma$).

5. Method according to any one of Claims 1 to 4, **characterized in that** the reference point (72, 72') is the corneal vertex of the eye (60, 62) of the subject (68) and/or **in that** the reference point (72, 72') is a fixed center of the pupil of the eye (60, 62) of the subject (68).

6. Method according to any one of Claims 1 to 5, **characterized in that** the position of the virtual pivot (55, 55') on the straight line (53g, 53g') fixed by the reference point (72, 72') with the known position in relation to the camera (36) or the spectacle frame (16) and the intersection (15, 15') of the viewing direction (17, 17') is determined by predetermining a fixed distance (E, E') of the virtual pivot (55, 55') from the reference point (72, 72').

7. Method according to any one of Claims 1 to 6, **characterized in that** the interpupillary distance $P_D$ of the eyes (60, 62) of the subject (68) is established by means of image analysis of an image acquired by the camera (36), said image containing the pupils of the eyes (60, 62) of the subject (68) and at least one portion of the spectacle frame (16) worn by the subject (68).

8. Method according to any one of Claims 1 to 7, **characterized in that** the angle δ which the optical axis (50) of the camera (36) forms with the perpendicular projection of the viewing direction (17, 17') in a vertical plane is established by means of image analysis of the acquired image of the at least one portion of the spectacle frame (16) worn by the subject (68).

9. Method according to any one of Claims 1 to 8, **characterized in that** the offset of the intersection Y of the distance line (54) of the pupils (56, 58) of the eyes (60, 62) with the perpendicular projection (50') of the optical axis (50) of the camera (36) into a horizontal plane (51'), in which the distance line (54) of the pupils (56, 58) of the eyes (60, 62) is situated, from the intersection Z of the distance line (54) of the pupils (56, 58) of the eyes (60, 62) with a vertical plane (52') which intersects the distance line (54) of the pupils (56, 58) of the eyes (60, 62) and which is perpendicular to the frame plane (69), the vertical axis of symmetry (74) of the spectacle frame (16) lying in said vertical plane, is established by means of image analysis of an image acquired by the camera (36).

10. Method for determining at least one fitting parameter from the group containing frame dimensions, the centration distance, interpupillary distance, lens blank diameter and pantoscopic tilt, **characterized by** determining a distance visual point (11, 11') according to any one of Claims 1 to 9 and determining the fitting parameters from the determined distance visual point (11, 11') and from additional information that is obtained from the acquired image by means of image evaluation.

11. System for determining a distance visual point (11, 11') on a spectacle lens (12, 14) which is receivable in a spectacle frame (16) and which has a frame plane (69), comprising
a camera (36), having an image plane (42), with an inclination sensor (44) detecting the inclination of the image plane (42) of the camera (36) about a horizontal axis (43), and
a computer unit (48) having
means for determining a pantoscopic angle α' of the spectacle frame (16) related to the position of the image plane (42),
means for correcting the pantoscopic angle α' of the spectacle frame (16) in accordance with the acquired inclination angle γ of the image plane (42) in relation to the vertical direction (46) so as to form a pantoscopic angle α related to the vertical direction (46),
means for determining a head rotation angle β of the head of the subject (68), formed by the optical axis (50) of the camera (36) with a plane perpendicular to the distance line (54) of the pupils (56, 58) of the eyes (60, 62) of the subject (68),
means for correcting the head rotation angle β in accordance with the detected inclination angle γ of the image plane (42) in relation to the vertical direction (46) so as to form a corrected head rotation angle β' corresponding to a horizontal alignment of the optical axis (50) of the camera (36), and
means for determining an intersection of the viewing direction (17, 17') of the subject (68) with the frame plane (69),
**characterized in that**
the computer unit (48) contains:

means for determining the distance visual point (11, 11') as the intersection of a virtual viewing direction (53, 53') lying in a horizontal plane with the frame plane (69),
by means of which the virtual viewing direction (53, 53') is determined as the direction of a virtual straight line (53v, 53v'), which is established by rotating a straight line (53g, 53g') which is set by a reference point (72, 72') arranged on the eye (60, 62) with a known position in relation to the camera (36) or the spectacle frame (16) and the intersection (15, 15') of the viewing direction (17, 17'),
by virtue of this straight line (53g, 53g') being, firstly, rotated at a virtual pivot (55, 55'), which lies at a distance from the reference point (72, 72') and on this straight line (53g, 53g') within the eye (60, 62), about an axis (46') lying parallel to the vertical direction (46) by a first angle $\beta_{OD}$", $\beta_{OS}$" dependent on the corrected head rotation angle β', and
by virtue of this straight line (53g, 53g') being, secondly, rotated at the virtual pivot (55, 55') about a further axis (47) lying parallel to the distance line (54) of the pupils (56, 58) of the eyes (60, 62) of the subject (68) by a further angle α" dependent on the corrected pantoscopic angle α, wherein, if the eye (60) is a right eye of the subject (68), the following applies for the first angle $\beta_{OD}$" which is dependent on the corrected head rotation angle β': $\tan \beta_{OD}$'' = $(0.5 \times P_D \times \cos \beta' + YZ) / (D + HSA)$,
and wherein, if the eye (62) is a left eye of the subject (68), the following applies for the first angle ($\beta_{OS}$" which is dependent on the corrected head rotation angle β': $\tan \beta_{OS}$'' = $(0.5 \times P_D \times \cos \beta' - YZ) / (D + HSA)$,
where $P_D$ is the interpupillary distance of the eyes of the subject,
where $D + HSA$ is the vertical distance of the camera (36) from a vertical plane (72), in which the distance line (54) of the pupils of the eyes (60, 62) is situated, and

where YZ is the offset of the intersection (Y) of the distance line (54) of the pupils (56, 58) of the eyes (60, 62) with the vertical projection (50') of the optical axis (50) of the camera (36) into a horizontal plane (51'), in which the distance line (54) of the pupils (56, 58) of the eyes (60, 62) is situated, from the intersection (Z) of the distance line (54) of the pupils (56, 58) of the eyes (60, 62) with a vertical plane (52') intersecting the distance line (54) of the pupils (56, 58) of the eyes (60, 62), said plane being perpendicular to the frame plane (69) and the vertical axis of symmetry (74) of the spectacle frame (16) lying in said plane; and

wherein the following applies for the further angle α" dependent on the corrected pantoscopic angle α:

$$\alpha'' = -(\gamma + \delta),$$

where δ is the angle which the optical axis (50) of the camera (36) forms with the perpendicular projection of the viewing direction (17, 17') into a vertical plane, in which the optical axis (50) of the camera (36) lies.

12. Device for determining at least one fitting parameter from the group containing frame dimensions, the centration distance, interpupillary distance, lens blank diameter and pantoscopic tilt using a system according to Claim 11, **characterized by** means for determining the fitting parameter from the distance visual point (11, 11') and from the captured image by means of image evaluation.

13. System according to Claim 11 or device according to Claim 12, **characterized in that** the camera (36) is integrated into a tablet computer (34) or into a cellular telephone, and the computer unit (48) is embodied as a server connected to the tablet computer or to the cellular telephone.

14. System according to Claim 13, **characterized in that** the tablet computer (34) or the cellular telephone communicates wirelessly with the server.

15. Computer program having program code for carrying out all method steps of a method according to any one of Claims 1 to 10 when the computer program is loaded into a computer unit or executed in a computer unit.

## Revendications

1. Procédé destiné à déterminer un point de vision de loin (11, 11') sur un verre de lunettes (12, 14) pouvant être installé sur une monture de lunettes (16) qui a un plan de monture (69),

dans lequel une image, située dans un plan image (42), d'au moins un secteur d'une monture de lunettes (16) portée par une personne de test (68) est détectée par une caméra (36) ayant un axe optique (50) qui contient les pupilles (56, 58) des yeux (60, 62) de la personne de test (68), tandis que la personne de test (68) regarde vers la caméra (36) avec une direction du regard (17, 17') d'au moins un oeil (60, 62) traversant le plan (69) de la monture,

dans lequel un angle d'inclinaison vers l'avant α', par rapport à la position du plan image (42), de la monture de lunettes (16) est déterminé, qui est corrigé en fonction de l'angle d'inclinaison γ détecté du plan image (42) par rapport à la direction verticale (46) en un angle d'inclinaison vers l'avant α par rapport à la direction verticale (46),

dans lequel un angle de rotation β de la tête de la personne de test (68), qui est formé par l'axe optique (50) de la caméra (36) avec un plan (52) perpendiculaire à la droite d'écartement (54) des pupilles (56, 58) des yeux (60, 62) de la personne de test (68) est déterminé, dans lequel l'angle de rotation β de la tête est corrigé conformément à l'angle d'inclinaison γ détecté du plan image (42) par rapport à la direction verticale (46) en un angle de rotation de la tête corrigé β' correspondant à une orientation horizontale de l'axe optique (50) de la caméra (36), et

dans lequel le point de vision de loin (11, 11') est déterminé par une analyse de l'image située dans le plan image (42) en tenant compte de l'angle de rotation de la tête corrigé β' correspondant à une orientation horizontale de l'axe optique (50) de la caméra (36), et dans lequel le point d'intersection (15, 15') de la direction du regard (17, 17') avec le plan (69) de la monture est déterminé au moyen d'une évaluation d'image ;

**caractérisé en ce que**

le point de vision de loin (11, 11') est déterminé comme étant l'intersection d'une direction du regard virtuelle (53, 53') se situant dans un plan horizontal (51) avec le plan de monture (69), dans lequel

la direction du regard virtuelle (53, 53') est déterminée comme étant la direction d'une droite virtuelle (53v, 53v') qui est déterminée en faisant tourner une droite (53g, 53g') définie par un point de référence (72, 72') de position connue sur l'oeil (60, 62) par rapport à la caméra (36) ou à la monture de lunettes (16) et l'intersection (15, 15') de la direction du regard (17, 17'),

en faisant tourner ladite droite (53g, 53'g) d'un premier angle $\beta_{OD}$", $\beta_{OS}$", qui dépend de l'angle de rotation de la tête corrigé $\beta$', autour d'un axe (46') parallèle à la direction verticale (46) vers un point de rotation virtuel (55, 55') espacé du point de référence (72, 72') et situé à l'intérieur de l'oeil (60, 62) sur ladite droite (53g, 53'g), et

en faisant tourner ladite droite (53g, 53g') au point de rotation virtuel (55, 55') d'un autre angle $\alpha$", qui dépend de l'angle d'inclinaison vers l'avant corrigé $\alpha$, vers l'autre axe (47) parallèle à la droite d'écartement (54) des pupilles (56, 58) des yeux (60, 62) de la personne de test (68),

dans lequel le premier angle $\beta_{OD}$", qui dépend de l'angle de rotation de la tête corrigé $\beta$', lorsque l'oeil (60) est un oeil droit de la personne de test (68), est donné par :

$\tan \beta_{OD}$" = (0,5 x $P_D$ x $\cos\beta$' + YZ)/(D + HSA),

et dans lequel le premier angle $\beta_{OD}$", qui dépend de l'angle de rotation de la tête corrigé $\beta$', lorsque l'oeil (62) est un oeil gauche de la personne de test (68) est donné par : $\tan \beta_{OS}$" = (0,5 x $P_D$ x $\cos\beta$' - YZ)/(D + HSA), où $P_D$ est la distance pupillaire des yeux de la personne de test (68),

où D + HSA est la distance verticale de la caméra (36) par rapport à un plan vertical (70) dans lequel se trouve la droite d'écartement (54) des pupilles des yeux (60, 62), et

où YZ est le déplacement du point d'intersection (Y) de la droite d'écartement (54) des pupilles (56, 58) des yeux (60, 62) avec la projection verticale (50') de l'axe optique (50) de la caméra (36) dans un plan horizontal (51') dans lequel se trouve la droite d'écartement (54) des pupilles (56, 58) des yeux (60, 62), par rapport au point d'intersection (Z) de la droite d'écartement (54) des pupilles (56, 58) des yeux (60, 62) avec un plan vertical (52') coupant la droite d'écartement (54) des pupilles (56, 58) des yeux (60, 62) et perpendiculaire au plan de montage (69) dans lequel se trouve l'axe de symétrie vertical (74) de la monture de lunettes (16), et

dans lequel l'autre angle $\alpha$", qui dépend de l'angle d'inclinaison vers l'avant corrigé ($\alpha$), est donné par :

$$\alpha" = -(\gamma + \delta),$$

où $\delta$ est l'angle formé par l'axe optique (50) de la caméra (36) avec la projection verticale de la direction du regard (17, 17') dans un plan vertical dans lequel se trouve l'axe optique (50) de la caméra (36).

**2.** Procédé selon la revendication 1, **caractérisé en ce que** l'angle d'inclinaison vers l'avant $\alpha$' de la monture de lunettes (16) par rapport à la position du plan image (42) est déterminé par analyse d'image de l'image détectée de l'au moins un secteur de la monture de lunettes (16) portée par la personne de test (68) et/ou **en ce que** l'angle de rotation $\beta$ de la tête de la personne de test (68), formé par l'axe optique (50) de la caméra (36) avec un plan perpendiculaire à la droite d'écartement (54) des pupilles (56, 58) des yeux (60, 62) de la personne de test (68), est déterminé par analyse d'image de l'image détectée de l'au moins un secteur de la monture de lunettes (16) portée par la personne de test (68).

**3.** Procédé selon la revendication 1 ou 2, **caractérisé en ce que** l'angle d'inclinaison vers l'avant corrigé $\alpha$ satisfait la relation suivante : $\alpha = \alpha$' - $\gamma$.

**4.** Procédé selon l'une des revendications 1 à 3, **caractérisé en ce que** l'angle de rotation de la tête corrigé $\beta$' satisfait à la relation suivante : $\beta$' = $\beta$/cos ($\alpha$' - $\gamma$).

**5.** Procédé selon l'une des revendications 1 à 4, **caractérisé en ce que** le point de référence (72, 72') est l'apex cornéen de l'oeil (60, 62) de la personne de test (68) et/ou **en ce que** le point de référence (72, 72')· est un centre fixé de la pupille de l'oeil (60, 62) de la personne de test (68).

**6.** Procédé selon l'une des revendications 1 à 5, **caractérisé en ce que** la position du point de rotation virtuel (55, 55') sur la droite (53g, 53g') définie par le point de référence (72, 72') de position connue par rapport à la caméra (36) ou à la monture de lunettes (16) et le point d'intersection (15, 15') de la direction du regard (17, 17') est déterminée en spécifiant une distance fixe (E, E') du point de rotation virtuel (55, 55') par rapport au point de référence (72, 72').

**7.** Procédé selon l'une des revendications 1 à 6, **caractérisé en ce que** la distance pupillaire $P_D$ des yeux (60, 62) de la personne de test (68) est déterminée par analyse d'image d'une image détectée au moyen de la caméra (36), qui contient les pupilles des yeux (60, 62) de la personne de test (68) et au moins un secteur de la monture de lunettes (16) portée par la personne de test (68) .

8. Procédé selon l'une des revendications 1 à 7, **caractérisé en ce que** l'angle δ formé par l'axe optique (50) de la caméra (36) avec la projection verticale de la direction du regard (17, 17') dans un plan vertical est déterminé par analyse d'image de l'image détectée de l'au moins un secteur de la monture de lunettes (16) portée par la personne de test (68).

9. Procédé selon l'une des revendications 1 à 8, **caractérisé en ce que** le déplacement du point d'intersection Y de la droite d'écartement (54) des pupilles (56, 58) des yeux (60, 62) avec la projection verticale (50') de l'axe optique (50) de la caméra (36) dans un plan horizontal (51') dans lequel se trouve la droite d'écartement (54) des pupilles (56, 58) des yeux (60, 62), par rapport au point d'intersection Z de la droite d'écartement (54) des pupilles (56, 58) des yeux (60, 62) avec un plan vertical (52') coupant la droite d'écartement (54) des pupilles (56, 58) des yeux (60, 62) et perpendiculaire au plan de montage (69) dans lequel se trouve l'axe de symétrie vertical (74) de la monture de lunettes (16), est déterminé par analyse d'image de l'image détectée par la caméra (36).

10. Procédé destiné à déterminer au moins un paramètre d'ajustement appartenant au groupe comprenant la dimension de la monture, la distance du point de centrage, la distance de la pupille, le diamètre du verre brut, l'inclinaison vers l'avant de la monture, **caractérisé par** la détermination d'un point de vue de loin (11, 11') selon l'une des revendications 1 à 9 et par la détermination du paramètre d'ajustement parmi le point de vision de loin (11, 11') déterminé et des informations supplémentaires obtenues à partir de l'image détectée par évaluation d'image.

11. Système destiné à déterminer un point de vision de loin (11, 11') sur un verre de lunettes (12, 14) pouvant être installé sur une monture de lunettes (16) qui a un plan de monture (69), comprenant :

une caméra (36) ayant un plan image (42), comprenant un capteur d'inclinaison (44) détectant l'inclinaison du plan image (42) de la caméra (36) autour d'un axe horizontal (43), et
une unité de calcul (48) comportant
des moyens destinés à déterminer un angle d'inclinaison vers l'avant α' de la monture de lunettes (16) par rapport à la position du plan image (42),
des moyens destinés à corriger l'angle d'inclinaison vers l'avant α' de la monture de lunettes (16) conformément à l'angle d'inclinaison γ détecté du plan image (42) par rapport à la direction verticale (46) en un angle d'inclinaison vers l'avant α par rapport à la direction verticale (46),
des moyens destinés à déterminer un angle de rotation β de la tête de la personne de test (68), formé par l'axe optique (50) de la caméra (36) avec un plan perpendiculaire à la droite d'écartement (54) des pupilles (56, 58) des yeux (60, 62) de la personne de test (68),
des moyens destinés à corriger l'angle de rotation β de la tête conformément à l'angle d'inclinaison γ détecté du plan image (42) par rapport à la direction verticale (46) en un angle de rotation de la tête corrigé β' correspondant à une orientation horizontale de l'axe optique (50) de la caméra (36), et
des moyens destinés à déterminer un point d'intersection de la direction du regard (17, 17') de la personne de test (68) avec le plan (69) de la monture,
**caractérisé en ce que**
l'unité de calcul (48) comporte :

des moyens destinés à déterminer le point de vision de loin (11, 11') comme étant le point d'intersection d'une direction du regard virtuelle se situant dans un plan horizontal (53, 53') avec le plan de montage (69), par lesquels la direction du regard virtuelle (53, 53') est déterminée comme étant la direction d'une droite virtuelle (53v, 53v') déterminée en faisant tourner une droite (53g, 53g') définie par un point de référence (72, 72') de position connue sur l'oeil (60, 62) par rapport à la caméra (36) ou à la monture de lunettes (16) et le point d'intersection (15, 15') de la direction du regard (17, 17'),
en faisant tourner ladite droite (53g, 53g') d'un premier angle $\beta_{OD}''$, $\beta_{OS}''$ dépendant de l'angle de rotation de la tête corrigé β', autour d'un axe (46') parallèle à la direction verticale (46) vers un point de rotation virtuel (55, 55') espacé du point de référence (72, 72') et situé à l'intérieur de l'oeil (60, 62) sur ladite droite (53g, 53g'), et
en faisant tourner ladite droite (53g, 53g') au point de rotation virtuel (55, 55') d'un autre angle α'' dépendant de l'angle d'inclinaison vers l'avant α corrigé, vers l'autre axe (47) parallèle à la droite d'écartement (54) des pupilles (56, 58) des yeux (60, 62) de la personne de test (68),
dans lequel le premier angle $\beta_{OD}''$, qui dépend de l'angle de rotation de la tête corrigé β', lorsque l'oeil (60) est un oeil droit de la personne de test (68), est donné par :

$$\tan \beta_{OD}" = (0,5 \times P_D \times \cos\beta' + YZ)>(D + HSA),$$

et dans lequel le premier angle $\beta_{OS}"$, qui dépend de l'angle de rotation de la tête corrigé $\beta'$, lorsque l'oeil (62) est un oeil gauche de la personne de test (68) est donné par : $\tan \beta_{OS}" = (0,5 \times P_D \times \cos\beta' - YZ)/(D + HSA)$, où $P_D$ est la distance pupillaire des yeux de la personne de test (68), où D + HSA est la distance verticale de la caméra (36) par rapport à un plan vertical (70) dans lequel se trouve la droite d'écartement (54) des pupilles des yeux (60, 62), et où YZ est le déplacement du point d'intersection (Y) de la droite d'écartement (54) des pupilles (56, 58) des yeux (60, 62) avec la projection verticale (50') de l'axe optique (50) de la caméra (36) dans un plan horizontal (51') dans lequel se trouve la droite d'écartement (54) des pupilles (56, 58) des yeux (60, 62), par rapport au point d'intersection (Z) de la droite d'écartement (54) des pupilles (56, 58) des yeux (60, 62) avec un plan vertical (52') coupant la droite d'écartement (54) des pupilles (56, 58) des yeux (60, 62) et perpendiculaire au plan de montage (69) dans lequel se trouve l'axe de symétrie vertical (74) de la monture de lunettes (16) ; et

dans lequel l'autre angle $\alpha"$, qui dépend de l'angle d'inclinaison vers l'avant corrigé $\alpha$, est donné par :

$$\alpha" = -(\gamma + \delta),$$

où $\delta$ est l'angle formé par l'axe optique (50) de la caméra (36) avec la projection verticale de la direction du regard (17, 17') dans un plan vertical dans lequel se trouve l'axe optique (50) de la caméra (36).

**12.** Dispositif destiné à déterminer au moins un paramètre d'ajustement appartenant au groupe comprenant la dimension de la monture, la distance du point de centrage, la distance de la pupille, le diamètre du verre brut, l'inclinaison vers l'avant de la monture, au moyen d'un système selon la revendication 11, **caractérisé par** des moyens destinés à déterminer le paramètre d'ajustement à partir du point de vision de loin (11, 11') et à partir de l'image détectée par évaluation d'image.

**13.** Système selon la revendication 11 ou dispositif selon la revendication 12, **caractérisé en ce que** la caméra (36) est intégrée à un ordinateur tablette (34) ou à un téléphone mobile et **en ce que** l'unité de calcul (48) est réalisée sous la forme d'un serveur connecté à l'ordinateur tablette ou au téléphone mobile.

**14.** Système selon la revendication 13, **caractérisé en ce que** l'ordinateur tablette (34) ou le téléphone mobile communique sans fil avec le serveur.

**15.** Programme d'ordinateur comportant un code de programme permettant la mise en oeuvre de toutes les étapes selon l'une quelconque des revendications 1 à 10 lorsque le programme d'ordinateur est chargé dans une unité de calcul ou exécuté dans une unité de calcul.

Fig.1

EP 3 304 183 B1

Fig.2

Fig.3

Fig.4

Fig.5a

Fig.5b

Fig.6a

Fig.6b

Fig.7a

Fig.7b

**IN DER BESCHREIBUNG AUFGEFÜHRTE DOKUMENTE**

**In der Beschreibung aufgeführte Patentdokumente**

- WO 2015107063 A1
- WO 0184222 A1
- US 20100195045 A1
- US 2010128220 A
- US 2010195045 A
- US 2015198822 A
- DE 102014200637
- DE 102004063981 B4